# EUROPEAN PATENT SPECIFICATION

(11) **EP 3 555 778 B1**
(45) Date of publication and mention of the grant of the patent: **21.10.2020**
(21) Application number: 16825349.0
(22) Date of filing: 15.12.2016
(51) Int. Cl.: G16H 10/60, A61B 5/026, G06T 17/00, G16H 20/40, G16H 30/20, G16H 50/20, G16H 50/50

(54) **METHOD AND PROCESS FOR PROVIDING A SUBJECT-SPECIFIC COMPUTATIONAL MODEL USED FOR DECISION SUPPORT AND MAKING DIAGNOSIS OF CARDIOVASCULAR DISEASES**
VERFAHREN UND VERFAHREN ZUR BEREITSTELLUNG EINES SUBJEKTSPEZIFISCHEN RECHENMODELLS ZUR ENTSCHEIDUNGSUNTERSTÜTZUNG UND DURCHFÜHRUNG EINER DIAGNOSE VON KARDIOVASKULÄREN KRANKHEITEN
PROCÉDÉ ET PROCESSUS DE FOURNITURE D'UN MODÈLE DE CALCUL SPÉCIFIQUE À UN SUJET UTILISÉ POUR L'AIDE À LA DÉCISION ET POUR LE DIAGNOSTIC DE MALADIES CARDIOVASCULAIRES

(43) Date of publication of application: 23.10.2019
(73) Proprietor: Sintef TTO AS, 7465 Trondheim (NO)
(72) Inventor: DAHL, Sigrid, Kaarstad, 7032 Trondheim (NO); URHEIM, Stig, 0374 Oslo (NO); SKJETNE, Paal, 7040 Trondheim (NO); MORUD, John Christian, 7041 Trondheim (NO); ZORIC, Josip, 7026 Trondheim (NO)
(74) Representative: Onsagers AS
(86) International application number: PCT/EP2016/081175
(87) International publication number: WO 2018/108276

(56) References cited:
- WO-A1-2014/097063
- JP-A- 2012 024 582
- US-A1- 2010 070 249
- US-A1- 2010 280 352
- US-A1- 2011 060 576
- US-A1- 2012 041 318
- US-A1- 2012 259 594
- US-A1- 2012 296 609
- US-A1- 2013 197 884
- US-A1- 2013 246 034
- US-A1- 2016 228 190
- US-B1- 8 548 778

## Description

### TECHNICAL FIELD

The invention relates to a method for providing a subject-specific computational model of at least one component in the cardiovascular system for simulating blood flow and/or structural features. The provided model can be used for providing decision support for enabling diagnosis that is more precise as well as for treatment of cardiovascular diseases including production and replacement of customized devices for the cardiovascular system.

### BACKGROUND

Cardiovascular disease (CVD) including valvular heart disease (VHD) is referred to as the next cardiac epidemic due to an imminent increase in the elderly population expected over the next decades. Assuming a prevalence of VHD of about 10% in the age group above 65, more than 10 million new cases of moderate to severe VHD will appear in Europe by 2030. Mitral valve regurgitation (MVR) accounts for approximately one third of all valvular heart diseases and the optimal treatment is valve repair. However, about 10-15% of patients suffer from failures 5-10 years after surgery. Failures have a substantial impact both for the individual patient that may need re-operation with higher morbidity and mortality and increased health costs to the society. The final decision of treatment alternatives based on expert judgment, and whether to perform valve repair or replacement might be highly subjective. Both the number and proportion of repairs relative to replacements may differ significantly from one heart centre to the other. This is also the issue when it comes to the type of reconstruction, as few studies are available and there is a lack of evidence-based recommendations.

The heart is responsible for pumping blood through the circulatory system. The anatomy and physiology of the heart have been studied thoroughly over the past years.

The system is split into two separate circuits, called the systemic and the pulmonary circuit. The heart can be seen as a double pump, where the right side of the heart pumps deoxygenated blood into the pulmonary circulation and the left heart pumps oxygenated blood through systemic circulation.

In the pulmonary circle, deoxygenated blood enters the right atrium (RA) from the venae cavae, from here it flows into the right ventricle (RV) which contracts and forces the blood through the pulmonary arteries and into the lungs. Blood in the lungs gets oxygenated before it returns to the left atrium (LA) via the pulmonary veins (PVs). The blood has now entered the systemic cycle. The left atrium guides the blood into the left ventricle (LV) which is the most powerful chamber. The ventricle ejects the blood into the aorta, which then distributes the blood throughout the body via a network of blood vessels, before the venae cavae bring the blood back into the right atrium where the process restarts.

### The cardiac cycle

The sequence of events that occur in the heart during one heartbeat is called the cardiac cycle. The events occur nearly simultaneously for the right and left heart. The typical resting heart rate in adults is 60 - 90 beats per minute (bpm). A physically fit person has a lower heart rate as compared to an inactive person.

Each heartbeat is commonly divided into two main phases: systole and diastole. Systole and diastole are synonymous with the contraction and relaxation of a heart muscle, respectively. Both the atria and the ventricles go through these two stages every heartbeat, but when we refer to the terms diastole and systole alone, we often mean the ventricular ones.

To analyse the events in more detail, the cardiac cycle can be divided into several stages. From a ventricular view, seven phases can be considered:
Phase 1: Atrial contraction; Phase 2: Isovolumetric contraction; Phase 3-4: Rapid and reduced ejection; Phase 5: Isovolumetric relaxation; Phase 6-7: Rapid and reduced filling.

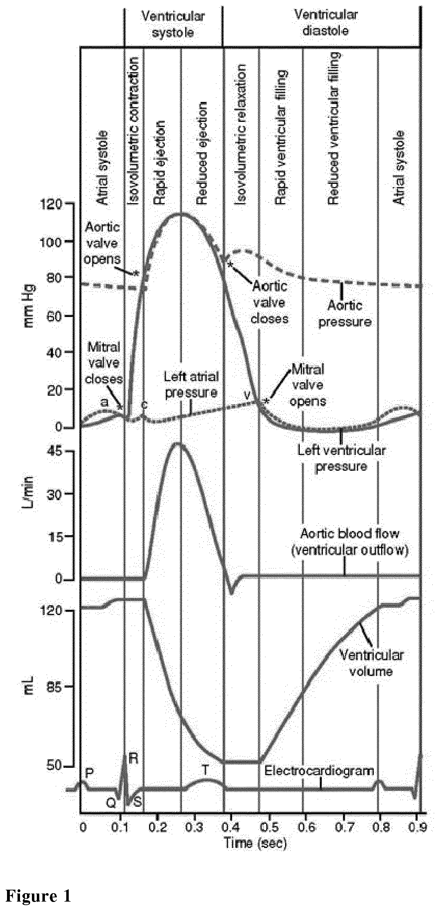

Timing of the events can be seen in Fig. 1 showing timing of various events occurring in the left heart during one cardiac cycle as adapted from Rooke and Sparks Jr. [T. W. Rooke and H. V. Sparks Jr. The Cardiac Pump, chapter 14, pages 237-251. Lippincott; Williams & Wilkins, 2003].

### The mitral apparatus

The mitral valve, also called the bicuspid valve, requires all its components in order to work properly. The components are the mitral annulus, the two mitral valve leaflets, the papillary muscles (PMs) and the chordae tendineae (abbreviated chordae), together they are called the mitral apparatus. The PMs and the chordae are known as the subvalvular apparatus.

The mitral annulus is a ring of fibrous tissue, which surrounds and supports the mitral orifice and anchors the two leaflets. The normal mitral valve orifice area in vivo ranges from 5.0 to 11.4 cm² (mean 7.6 ± 1.9 cm²). The shape of the annulus approximates a hyperbolic paraboloid, often described as a three-dimensional saddle. Studies indicate that the saddle shape of the annulus might play an important role in optimizing chordal force distribution and reducing leaflet stress.

The mitral valve consists of two leaflets; the anterior and the posterior leaflet. Their size and circumferential length are quite different. The anterior leaflet is adjacent to the aortic artery and occupies one third of the annular circumference, whereas the posterior leaflet occupies the rest.

The anterior leaflet is largest, and the leaflet is actually big enough to cover the mitral orifice alone. The posterior leaflet has a more supporting role and its movement is more restrained by the tendinous cords. During ventricular filling the soft leaflets comply and fold into the ventricle, allowing blood to pass freely. During ventricular contraction, the valve closes; the leaflets will then fold towards each other, forming a seal. This seal is called the coaptation zone. When the coaptation length is more than 7-8 mm, measured from the tip to the point where the coaptation ends, the valve is usually competent and there will be no regurgitation.

The subvalvular apparatus, consisting of the papillary muscles and the chordae tendineae, lies completely in the left ventricle (LV).

There are two papillary muscles in the LV; the anterolateral one and the posteromedial one. The PMs are cone-shaped muscles extending upward from the ventricular free wall and into the LV cavity. The chordae tendineae are string-like fibrous structures, which terminate from the tip of the PMs and insert into the ventricular surface of the mitral leaflets. Both of the PMs have chordae attachments to both of the leaflets. The chordae divide into branches. There are approximately between 15 and 32 major chordal trunks arising from the PMs, on the other end, approximately 100 individual cords are attached to the two leaflets.

The main function of the subvalvular apparatus is to prevent the valve leaflets from being everted into the atrium when the ventricle contracts. During systole, the PMs contract to tighten the chordae tendineae. The forces exerted by the leaflets on the cords are then transferred to the PMs, hence they have an essential role in load bearing of the mitral valve during LV contraction. The distance between the PMs tips and the mitral annulus is approximately constant during systole. During diastole, the PMs relax and elongate.

### Blood flow

Many attempts have been made in order to develop a general constitutive equation for blood. However, a theoretical reliable model covering all relevant regimes of physiological blood flow still does not exist. The study of the movements of blood and of the forces concerned is often referred to as hemodynamics.

Blood is a multipart medium consisting of cells and cell fragments suspended in a liquid. The liquid is called plasma and makes up about 55% of the total blood volume. Plasma is composed of 91.5% water, 7% proteins and 1.5% other solutes. The remaining 45% of the blood volume consists of different blood cells, also called hematocytes. The three main kinds of hematocytes are the red blood cells (RBCs), the white blood cells (WBCs) and cell fragments called platelets. Under physiological conditions the WBCs and the platelets occupy only 1/600th and 1/800th of the total cell volume, respectively, i.e. the RBCs accounts for the major part of the cellular blood volume. The volume fraction of RBCs in whole blood is called the hematocrit level.

Plasma alone behaves like a Newtonian fluid with a dynamic viscosity of 1.2·10⁻³ kg/(m·s) at 37 °C. The term "dynamic viscosity" refers to the property that characterizes the frictional resistance of a fluid to flow. However, due to the high cellular content, the whole blood behaves like a non-Newtonian fluid. The cardiovascular system is a network of vessels with geometries varying from the smallest vessels in the capillary network to the large heart chambers. It is therefore useful to characterize hemodynamic properties in terms of the environment in which the blood flows. In the smallest vessels, the inner diameter is about the same size as the RBCs, ranging from 4 to 8µm. When blood flows through these vessels, the RBCs have to be squeezed and deformed and move in single file. The blood can then be characterized as highly non-Newtonian. However, in the largest arteries and in the heart chambers, the non-Newtonian effects are weak because of the large dimensions. The blood can then be considered as a homogeneous fluid with Newtonian properties i.e. a constant coefficient of viscosity. The dynamic viscosity of blood in large vessels at normal physiological conditions is 3.5·10⁻³ kg/(m·s). Another common assumption is that blood is an incompressible fluid. The assumption of incompressibility comes from the fact that the density is unaffected by the pressure in the range of pressure concerned in physiology. The density is assumed to be in the range 1050-1060 kg/m³ depending mainly on haematocrit.

In order to improve patency of a repair or replacement of a heart component, e.g. a heart valve, there is a need for a reliable and accurate method for providing a computational model of at least one heart component for simulating blood flow and/or structural features for a specific person. In this disclosure, such a model is called a subject-specific computational model.

Biomechanical problems are most often multidisciplinary and can involve elements from several domains like fluid mechanics, structural mechanics, electro-mechanics, scientific computing, mathematical modelling etc.

Computational cardiac modelling and simulation are no exception. The heart is a highly complex organ where the flow, structural and electrical phenomena are tightly coupled. Electrical signals trigger mechanical activation, the heart walls contract and blood is ejected into the pulmonary and systemic circuits. However, this is not a one-way system, the blood flow influences the vessel wall mechanics and the deformation of the tissue again influences the electrical properties. A fully integrated model is the most promising tool for solving the overall heart function.

However, a fully coupled physiological model of the heart, which is clinically feasible, does still not exist. With the aim of getting into clinical research in a shorter time, separate approaches are currently more common. As such, the inventors have, among other things, focused on the hemodynamics in the left side of the heart.

The numerical techniques used for solving problems involving fluid flows are often referred to as computational fluid dynamics or CFD. Different approaches have been suggested in order to create CFD models of cardiac blood flow. All studies have various shortcomings and only a few models can be applied on a clinical basis. Some aspects of cardiac CFD simulations are presented and briefly discussed.

### CFD approaches

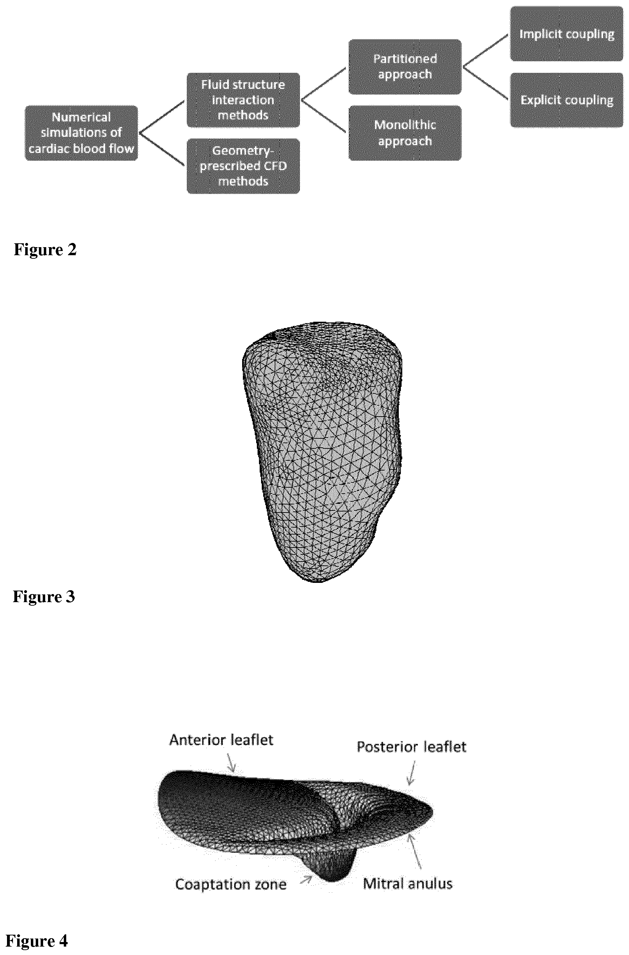

The numerical simulations of cardiac blood flow can roughly be classified into two main groups as illustrated in Fig. 2. First, fluid structure interaction or FSI models, which take into account the interaction between the fluid flow and the surrounding tissue. Second, geometry-prescribed CFD models, which uses prescribed wall movements as a boundary condition for the CFD simulation.

In a FSI simulation, the fluid flow exerts forces on the surrounding structure, the structure will then deform and in turn, affect the fluid flow. Hence, a FSI problem consists of a structural problem and a flow problem coupled together. The FSI problems can be solved using a monolithic or a partitioned approach.

Figure 2 is a flowchart illustrating that numerical simulations of cardiac blood flow can roughly be classified into two main groups. The flowchart also gives an overview of the main techniques used to solve the FSI problems.

In the monolithic approach, the structural equations and the flow equations are solved simultaneously using a single code. In the partitioned approach, the structural equations and the flow equations are solved within separate codes. The separate codes might be in-house codes or existing commercial solvers. The partitioned approach requires a coupling algorithm that can couple the fluid and the structural systems in a stable way and assure convergence within a reasonable amount of time.

As illustrated in Fig. 2, the partitioned approach can be further categorized as implicit or explicit coupling. In the implicit (also known as strongly coupled) partitioned techniques, iterations are performed within each time step until equilibrium between the fluid and the structure is achieved. In the explicit (also referred to as weakly or loosely coupled) partitioned techniques, the flow equations and the structural equations are solved only once or a fixed number of times within each time step. The lack of coupling iterations within each time step reduces the computational cost, but equilibrium is not necessarily achieved and the coupling scheme might become unstable. The explicit technique is therefore only sufficient when the interaction between solid and fluid is weak. If the interaction is strong, an implicit coupling technique is needed. The interaction is strong if for example the density ratio of fluid and structure is high, the fluid is incompressible or the structure is very flexible.

In a geometry-prescribed CFD simulation the boundary motion is known a priori. The geometry- prescribed CFD method is therefore a one-way approach, which does not consider the interaction with the structure. This simplifies the modelling because there is no need for a material model or a numerical scheme capable of simulating the coupled system. However, a deforming geometry and a CFD solver capable of handling the large deformations of the fluid domain are necessary.

### Deforming fluid domain

Traditionally, CFD simulations have been performed in domains, which do not deform. In biomechanical problems, this is often not the case. There exist several techniques for calculating the flow equations in a deforming fluid domain e.g. the fixed grid methods, the moving grid methods and the mesh free methods.

The fixed grid method is a non-boundary fitted method. The influence of the structure is introduced by momentum sources in the momentum equations of the flow. The first non-boundary fitted method was proposed by Peskin and is now known as the immersed boundary method, [C. S. Peskin and D. M. McQueen. A three-dimensional computational method for blood flow in the heart. 1. immersed elastic fibers in a viscous incompressible fluid. Journal of Computational Physics, 81:372-405, April 1989*.].* Another similar approach is the fictitious domain method described by Glowinski et al. [R. Glowinski, T.-W. Pan, and J. Periaux. A fictitious domain method for dirichlet problem and applications. Computer Methods in Applied Mechanics and Engineering, 111:283-303, 1994*.].* The fictitious domain method can be seen as the finite element (FE) version of the immersed boundary method, which is developed within the finite difference (FD) framework.

An advantage of the fixed grid methods is that the flow solver can be simple and fast because the fluid grid does not have to deform. However, a major drawback have been the loss of accuracy near the fluid-structure interface.

The moving grid method is a boundary fitted method where the fluid mesh moves with the moving interface throughout the computation. A common technique is to use the Arbitrary Lagrangian- Eulerian (ALE) formulation to express the Navier-Stokes equations on the moving grid. In the Eulerian formulation, the grid is fixed and the material moves through it. In the Lagrangian formulation, every grid point moves at the same velocity as their associated material point. In the ALE-formulation the grid and the material can move at different velocities. In other words, the grid can deform at an arbitrary velocity and not necessarily at the velocity of the fluid, hence the name. At the fluid-structure interface, the fluid grid follows the velocity of the structure. The resulting grid displacements at the interface will, in turn, be extended to the rest of the fluid domain by some mesh updating method.

In a major class of mesh free methods the fluid is discretized into Lagrangian elements/parcels with a given initial mass and density. The elements are then free to move relative to one another without any regard to a specific topology or connectivity. A popular example is so called Smoothed Particle Hydrodynamics (SPH) [Gingold R.A., Monaghan J.J.; Smoothed particle hydrodynamics - Theory and application to non-spherical stars. Monthly Notices of the Royal Astronomical Society, vol. 181, Nov. 1977, p. 375-389]*.* However, when solving for the motion of the elements additional effort is needed to keep track of the adjacent particles that the Lagrangian elements interact with in order to solve the momentum balance of the system. Interaction between the Lagrangian elements in mesh free methods and bounding geometry also needs additional attention. The methods show great promise in solving problems with dynamic boundaries [Kulp S.A, Ventricular blood flow simulation and analysis for cardiovascular diagnostics, PhD dissertation New Brunswick Rutgers, The State University of New Jersey, January 2015*].*

### Motivation of the invention

The objective of the invention is to use models and methods that contribute to a better understanding, description and prediction of cardiac blood flow. Thus, a Computational Fluid Dynamics (CFD) approach is provided for the heart chambers. To obtain physiologically representative models, the time-varying geometries can be rendered from medical imaging data.

A great advantage of the present invention is obtained by choosing the ALE-formulation, as the wall shear stress at the fluid-structure interface can be calculated accurately. This is important in cardiac CFD simulations, and constitute an advantage compared to prior art.

The present invention can be summarized as a subject-specific simulation model of the cardiovascular system and blood flow. The simulation model can for example be used as a tool for cardiovascular diagnostic and for evaluating alternative interventions. The invention also regards non-invasive medical imaging of the cardiovascular system making it possible to detect and grade pathology related to both anatomical and physiological abnormalities.

The subject-specific simulation model of a pumping heart, provided by the invention, is reconstructed by coupling CFD or Fluid Structure Interaction (FSI) algorithms with medical imaging, e.g. ultrasound, MRI or CT. Such models make it possible to understand the complex flow phenomenon and provide flow details on a level not possible by medical imaging alone.

The subject-specific models provided by the invention, is a tool for clinical decision-making, an objective support for health care professionals in diagnosing and in making decisions prior to surgery. As such, the simulation model provides new insight into the outcome of surgical intervention on cardiovascular blood flow.

### SHORT DESCRIPTION OF THE INVENTION

The subject-matter of present invention is disclosed in claims 1-23. The present invention is defined by a computer implemented method for providing a subject-specific computational fluid dynamic model (CFD) of at least one component in the cardiovascular system for simulating blood flow and/or structural features, wherein the model comprises transient geometry and is created by:- acquiring subject-specific measurement data of said at least one component, and- generating the computational fluid dynamic model based on the subject-specific measurement data, characherized in - acquiring the subject-specific measurement data by performing echocardiography in real-time 3D for creating time-dependent ultrasound measurements representing medical imaging data, and further defining at least one boundary condition or source term for the model from the transient geometry based on time-varying geometrical shapes of the medical imaging data when running a simulation.

In one embodiment of the invention, the method comprises using flow and/or pressure measurement data for acquiring flow and/or pressure specific data related to the at least one component in the cardiovascular system

In one embodiment of the invention, the method comprises generating the at least one boundary condition or source term by using time-dependent movement of the at least one component in the cardiovascular system, thereby determining a movement interval of the at least one component in the cardiovascular system.

In one embodiment of the invention, the at least one component in the cardiovascular system is a heart component.

In one embodiment of the invention, the method further comprises using the time-dependent movement of at least a part of a cardiac wall, cardiac volume, cardiac or prosthetic valves as the at least one or more components in the cardiovascular system for generating the at least one boundary condition or source term.

In one embodiment of the invention, the time-dependent movement of at least a part of the vascular wall, vascular volume or vascular valves is used for generating the at least one boundary condition or source term.

In one embodiment of the invention, the time-dependent movement of at least a cardiac pumping device is used as the at least one or more component in the cardiovascular system for generating the at least one boundary condition or source term.

In one embodiment of the invention, the boundary conditions is changed dynamically during a simulation cycle.

In one embodiment of the invention, the source terms is changed dynamically during a simulation cycle.

In one embodiment of the invention, the transient geometry is based on medical real-time imaging data.

In one embodiment of the invention, the blood flow is simulated by using a Computational Fluid Dynamics (CFD) method and/or Fluid Structure Interaction (FSI) method.

In one embodiment of the invention, the structural features of the model is simulated by Computational Structural Dynamics (CSD) and/or FSI method.

In one embodiment of the invention, the model is created by adding subject-specific data, such as hematological and/or tissue sampling, physicochemical data, or subject-specific metadata.

In one embodiment of the invention, the model is created by further inputting data related to one or more of the following: prosthetic heart valves, cardiac pumping devices, vascular devices or grafts.

In one embodiment of the invention, the model is created by data related to corrective surgical procedures.

One embodiment of the invention comprises collecting measurement data comprising medical data acquired from different imaging modalities such as Magnetic Resonance (MR), X-ray, Computational Tomography (CT), Positron Emission Tomography (PET) and ultrasonography.

One embodiment of the invention comprises creating the model by subject-specific data combined with non-subject-specific data of the cardiovascular system and components thereof.

One embodiment of the invention comprises arranging the model as a machine learning model for continuously optimizing treatment planning and / or decision making and / or for diagnostic purposes by inputting at least one of the following: prior simulation results, patient history, and pre-, peri- or post-operative effects.

One embodiment of the invention comprises arranging the model to choose which procedure to simulate based on one or more of the following: suggestions from the machine learning system; information of different cardiovascular devices and choices made by personnel and/or the patient.

The present invention is further defined by a process for clinical treatment planning and/or diagnostic purposes, in pre-, peri- or post-operative decision support using the subject-specific computational model obtained by the method described above.

In one embodiment of the invention, the process is being used for predicting the outcome of a medical procedure.

In another embodiment of the invention, the process is being used for designing optimized individual prostheses designs.

In yet another embodiment of the invention, the process is being used for designing subject-specific heart valves and/or cardiovascular devices.

### BRIEF DESCRIPTION OF THE DRAWINGS

Preferred embodiments of the subject matter described herein will now be explained in the detailed description with reference to the accompanying drawings.
Fig. 1 shows timing of various events occurring in the left heart during one cardiac cycle as adapted from Rooke and Sparks Jr.
Figure 2 is a flowchart illustrating that numerical simulation of cardiac blood flow can roughly be classified into two main groups;
Figure 3 shows a closed 3D surface mesh of the endocardial LV at one instance of the cardiac cycle.
Figure 4 shows a subject-specific 3D model of the physiological mitral valve at peak systole;
Figure 5 shows the complete subject-specific 3D model, *Cₛ*, of the LV including the MV and the proximal part of the ascending aorta (Aao) at different instances during a complete systole;
Figure 6 shows realignments of the 3D model with the original echocardiographic data for verifying the geometry.
Figure 7 shows the AML curvature with a defined angle θ;
Figure 8 shows modified MV models at different angles θ;
Figure 9 shows velocity streamlines in a long-axis view of the LV at different time steps;
Figure 10 illustrates the location of the cross sections PI to P5, where PI is at the level of the aortic annulus, P2 is located XX cm downstream of P1. The posterior and anterior side of the Aao is also depicted in the figure;
Figure 11 shows velocity contours at peak systole (100ms) at plane A1 and A3 for C₅, C₁₅, C₃₀ and C₆₀ respectively. Velocity range is 0 - 1.5 m/s;
Figure 12 shows velocity contours at 280ms at plane A1 and A3 for C₅, C₁₅, C₃₀ and C₆₀ respectively. Velocity range is 0 - 0.45 m/s;
Figure 13 shows examples of isosurfaces of λ_{2.} a): *C60,* λ₂ = -500, t = 285ms + velocity streamline in a section (about y=0) b): *Cₛ,* = -500, t = 285ms, c): *C₆₀*, λ₂ = -500, t = 285ms.

### DETAILED DESCRIPTION

Recent advances in medical imaging technologies generating vast amounts of data has opened for the present invention, describing a new method and process for providing a subject-specific computational model of at least one component in the cardiovascular system, e.g. a heart component, for simulating blood flow and/or structural features. This is made possible by handling and post-processing imaging data as input for generating the computational model that may be used for improving diagnosis and / or planning treatment of Cardiovascular Disease (CVD) including Valvular Heart Disease (VHD). The computational model can be used for avoiding failures risking higher morbidity and increased health costs to the society.

Furthermore, the new technologies have facilitated the invention as decision support and help in designing patient-specific designed devices, e.g. valves, made for individually optimized treatment involving operational procedures obtaining higher long-term survival and significantly reduced need for reoperations.

As mentioned, the objective of the invention is to use models and methods that contribute to a better understanding of cardiac blood flow and enable more precise diagnosis and treatment planning. Thus, a geometry-prescribed CFD approach is provided for the heart chambers. To obtain physiologically representative models, some of the time-varying geometries are rendered from medical imaging data.

### Definitions

The following is a list of definitions used throughout this disclosure.

The term "computational model" as used herein refers to mathematical model in computational science that makes it possible to study the behaviour of a complex system by computer simulation.

The term "simulation" as used herein refers to imitation of an operation of a real-world process or system over time.

The term "subject-specific" as used herein refers to something being adapted to a particular individual, either a patient or a healthy individual. The terms "subject-specific" and "patient-specific" are used interchangeably herein.

The term "non-subject-specific" as used herein refers to something being applicable to many individuals, like text-book examples and/or input data generated based on statistical analysis of features.

The term "heart component" as used herein refers to any part or component of the heart, such as valves, atria, ventricles, arteries, veins etc.

The term "blood flow" as used herein refers to the motion of blood, described by for example the velocity and pressure of the blood in time and space.

The term "structural feature" as used herein refers to the natural physical features of a biological structure.

The term "transient geometry" as used herein refers to time-varying geometrical shape, described by e.g. coordinate locations of walls as function of time.

The term "flow measurement" as used herein refers to using an instrument to record data from a fluid flow, such as recording velocities, pressures, turbulence levels, temperatures or other quantities.

The term "boundary conditions" as used herein refers to the conditions such as velocities, pressures, temperatures or similar at the border of the geometrical domain or at an internal interface inside the domain, such as inflow, outflow, walls etc. Also including conditions at solid - fluid interfaces both inside and at the border of the geometrical domain.

The term "time-dependent" as used herein refers to a situation that evolves with time but including as a special case situation that remain the same with time.

The term "movement interval" as used herein refers to motion during a time interval that is part of or the complete cardiac cycle.

The term "driving pressure" as used herein refers to a difference in pressure between two locations that can result in a motion.

The term "real-time" as used herein refers to digital signal processing (DSP) where input data is continuously analysed for generating output data in the time it takes to input and output the same set of samples independent of the processing delay.

The term "Computational Fluid Dynamics" (CFD) as used herein refers to the use of computers to predict the motion of fluids.

The term "Computational Structural Dynamics" (CSD) as used herein refers to the use of computers to predict the forces, deformations and / or motion of structures.

The term "Fluid Structure Interaction" (FSI) as used herein refers to the use of computers to predict the forces, deformations and / or motion involving both fluids and structures, including the combination of CFD with CSD.

The term "algorithm" as used herein refers to a recipe for performing a calculation or computation.

The term "blood/tissue sampling" as used herein refers to collecting blood (usually in a glass tube) and removal of tissue from any part of the cardiovascular system for further analysis in the laboratory.

The term "metadata" as used herein refers to data that provides information about other data. There are two types of metadata, i.e. structural metadata defining the structure of a data file, and descriptive metadata describing what the data represent. The purpose of metadata is to help users find relevant information and discover resources. Metadata also helps to organize electronic resources, provide digital identification, and support the archiving and preservation of resources.

The term "prosthetic" as used herein refers to any type of artificial part or component for use as a replacement for a natural body part or component, such as heart valves. As used herein the prosthesis may be made of biological material, such as pig valves, or it may be artificially constructed such as mechanical heart valves.

The term "heart device" as used herein refers to any device useful in treatment of heart disease, such as artificial annulus rings, stents etc. The term also includes devices that are useful in relation with heart surgery, i.e. surgical devices to be used during surgery.

The term "corrective surgical procedure" as used herein refers to a procedure with the aim of correcting the natural components or part of the cardiovascular system, e.g. bulging heart valves, para-valvular leakage etc.

The term "imaging modality" as used herein refers to any mode or form able to give imaging data and/or measurement data and/or metrics, such as, but not limited to, sonography, Magnetic Resonance (MR), X-ray, Computational Tomography (CT), and Positron Emission Tomography (PET).

The term "imaging data" as used herein refers to data obtained from measurement recordings from medical imaging instruments, modalitites and metrics, such as ultrasound, MR, CT, PET, and echocardiography or similar.

The term "medical imaging" as used herein refers to any measurement principle that can be used to provide imaging data and/or measurement data and/or metrics of the cardiovascular system,

The term "machine learning" as used herein refers to the ability of computers to learn without being explicitly programmed, i.e. a self-learning system. This is the subfield of computer science that evolved from the study of pattern recognition and computational learning theory in artificial intelligence and relies on algorithms that can learn from and make predictions on data.

The term "simulation result" as used herein refers to the result or outcome of a simulation, i.e. the predicted result based on the input data.

The term "pre-operative", "peri-operative" and "post-operative" as used herein refers to the period before, during and after an operation, respectively.

The term "decision support" as used herein refers to a recipe or computer program that can be used to aid or provide information and / or advice to support a decision and / or a decision-making process.

The term conservation equation as used herein refers to an equation that describes the evolution of a conserved quantity, such as mass, momentum or energy.

The term "source term" as used herein refers to a term in a conservation equation that represents the generation or destruction of a conserved quantity, such as a force in a conservation equation for momentum or the production of a chemical species in a conservation equation for mass.

### Aspects of the invention

According to one aspect of the disclosure from one or more imaging technologies are combined with prior knowledge of the physiology of a heart, as well as the vascular system. The invention concerns new improved algorithms for flow, pressure mapping and surface rendering. The invention may be employed together with database content and history matching to previous cases forming new pre-operative planning systems, possible re-evaluation during operation, and design of individually optimized procedures and personalized valves, e.g. the mitral valve. Further the invention concerns methods for assessing optimized positioning and orientation of the valve to reduce the risk of degeneration leading to the need for replacement and compensating for any shear stress on other tissue i.e. heart or valves or vessels.

The disclosure as such is mainly a tool for aiding experts/professionals in the diagnosis or detection of CVD and / or identifying the optimal treatment for each individual patient suffering from CVD, like for instance mitral valve disease. By supporting patients with patient-specific computational simulation models and/or history matching, a more precise diagnosis and / or prediction and evaluation of the outcome of alternative surgical techniques is possible. The new procedure creates an objective planning and decision support platform prior to surgical procedures.

A simulation environment offers flexible control of the boundary conditions and / or source terms (mimicking healthy and diseased myocardial tissue or vessel wall) and flow parameters (corresponding to changed hemodynamic loads). This gives the opportunity to easily alter the models and further check how flow responds to the applied changes. As such, the inventors of the present invention have created subject-specific models that have the potential to support professionals in clinical decision-making by performing virtual surgery on a specific subject/patient. By doing this, new insights into the impact of alternative surgical interventions on the blood flow can be obtained for each individual patient.

Furthermore, a new understanding of flow, pressure, tissue stress, and surgical options improves the operative results in general for any choice of valve or graft or other cardiovascular devices, and even better, when valves, grafts or other devices are redesigned for optimum outcome for each patient.

The simulation model according to the present disclosure can be used to choose type of valves or grafts, or redesign or repair or correct the present valves for an optimized outcome. The invention thus supplies a method for making, redesigning or repairing valves or grafts, which are able to reduce the stress on the overall heart tissue and on other valves, thus improving long-term survival and reducing the need for re-operation to a minimum.

The disclosure uses numerical solution of the Navier-Stokes equations.

In cardiac CFD simulations it can be an advantage with detailed information about the geometry and the time-varying motion of the heart chambers. In the geometry-prescribed CFD approach, an initial geometry and a prescribed boundary motion needs to be implemented in the model. In the FSI approach, an initial geometry and an appropriate material model for calculating the mechanical behaviour of e.g. the vessel wall is necessary. The FSI approach is promising, but currently no FSI method solves this problem with high accuracy. In one embodiment of the invention, a geometry-prescribed CFD method is thus applied to obtain a clinical in situ analysis and evaluation of cardiac flow. It is important to remember that the blood flow pattern will be the same whether the same wall motion is prescribed or computed by a coupled solution, thus both approaches are applicable.

In the different aspects of the disclosure, the geometries might be simplified and idealized or obtained from medical imaging. By simplifying the complex geometries to idealized models, valuable information might be lost. On the other hand, simplified models might be important pioneering steps on the way to more refined models. By rendering the geometries from medical imaging data, subject-specific CFD models are obtained. Subject-specific CFD simulations can provide flow details on a level not possible by medical imaging alone. Such quantitative information makes it possible to understand the complex flow phenomenon occurring under both normal and pathological conditions.

CFD simulations can be supplemented or replaced by simplified analytical expressions for flow velocities and pressures, such as the Bernoulli equation. This can be used for validation of CFD simulations, or to replace parts of the CFD analysis in the invention. This can be useful in e.g. real-time applications due to the low computational cost of the analytical approach.

One aim of the invention is to apply machine learning and artificial intelligence for all relevant cardiovascular diseases with data from both patients as well as healthy populations. By using multimodality image fusion and physiological data, it is possible to create a refined computational model for each individual case.

The computational model can represent at least one component in the cardiovascular system for simulating blood flow and/or structural features. For explaining the invention and the model, a heart is used as a typical example of at least one component in the cardiovascular system. The invention is however not restricted to simulating a heart. The model can be used for simulation other components comprised in the cardiovascular system.

A Computer Aided Workflow for Integrated Diagnostics and Personalized Treatment Planning is supplied. As such, the invention includes a new method for assessing abnormalities in the cardiovascular system and in the blood flow and enables a standardized and effective decision support system comprising several steps.

The present disclosure is more specifically defined by a method for providing a subject-specific computational model of at least one component in the cardiovascular system for simulating blood flow and/or structural features, wherein the model comprises transient geometry and is created by performing several steps. The first step is acquiring different types of subject-specific data of said at least one component. These data will provide a basis for the model and may in one embodiment of the invention comprise imaging data of the cardiovascular system acquired by means of e.g. ultrasound, MRI, CT, fluoroscopy.

In one embodiment the echocardiography is performed in real-time 3D for creating time-dependent ultrasound measurements.

This information is preferably co-processed with knowledge about heart physiology, when the at least one component in the cardiovascular system is a heart component, by using new image post-processing algorithms for flow and pressure calculations displayed in 0D, 1D, 2D, 3D or 4D presentations. These data form a basis for accurate calculation and image representation that are combined with 3D flow and pressure data for all heart structures, valves and vessels of importance for the optimum function of the diseased heart post-surgery, including also a better objective determination of which cases not to perform surgical procedures on.

The imaging data is linked to collected relevant information from the patients' medical record (age, body mass index, blood pressure, blood composition, other diseases, medication and so on).

Different types of subject-specific data of the at least one cardiovascular component may have to be converted to a common basis for use in the model. This will ensure that the data will have the same magnitude and meaning when being used for generating the computational model.

The next step of the method is generating the computational model based on the subject-specific data and letting the transient geometry of the model define at least one boundary condition or source term for the model when running a simulation. The transient geometry refers to time-varying geometrical shape. This may for instance be coordinate locations of walls in the model as function of time. Such coordinates can e.g. be used to specify the velocity boundary condition at the wall. In this way, the invention provides a method for creating subject-specific 3D boundary conditions or source terms for simulations of cardiac flow, based on subject-specific data obtainable by an imaging modality. The algorithms provide a framework for the coupling of different data sets of the pulmonary arteries, LA, LV, the LVOT, the MV and the subvalvular apparatus, the aortic valve and the first part of the ascending aorta.

In one aspect of the disclosure the method further comprises using flow and/or pressure measurement data for acquiring flow and/or pressure specific data related to the at least one component in the cardiovascular system. Such flow and/or pressure specific data can in some cases improve the accuracy of the simulation model and/or be necessary to run a subject-specific simulation. In other cases, such subject-specific data can be necessary to validate the simulation results.

In one aspect of the disclosure the at least one boundary condition or source term is generated by using time-dependent movement of the at least one component in the cardiovascular system, thereby determining a movement interval of the at least one component in the cardiovascular system. A movement interval could for example be the movement of the component in a part of the cardiac cycle, e.g. systole or a part of systole. The specific movement and the limits of the movement interval can be input to the simulation software.

One aspect of the disclosure is using the time-dependent movement of at least a part of a cardiac wall, cardiac volume, cardiac or prosthetic valves as the at least one or more components in the cardiovascular system for generating the at least one boundary condition or source term.

Another aspect of the disclosure is using the time-dependent movement of at least a part of a vascular wall, vascular volume or vascular valves for generating the at least one boundary condition or source term.

Yet another aspect of the disclosure is using the time-dependent movement of at least a cardiac pumping device as the at least one or more component in the cardiovascular system for generating the at least one boundary condition or source term.

The at least one component in the cardiovascular system can be any part. In one embodiment of the invention, the component is a heart.

In one aspect of the disclosure the boundary conditions and/or source terms change dynamically during a simulation cycle. Meaning that each boundary condition and/or source terms could be time-dependent and / or applied and / or valid in whole or only in specific time intervals of the simulation.

In one aspect of the disclosure the transient geometry is based on medical imaging data, e.g. real-time ultrasound recordings.

In one embodiment of the invention, the blood flow is simulated by a using Computational Fluid Dynamics (CFD) method and/or Fluid Structure Interaction (FSI) method.

In one embodiment of the invention, the structural features of the model are simulated by Computational Structural Dynamics (CSD) and/or FSI method.

In one aspect of the disclosure the method comprises creating the model by inputting additional subject-specific data, such as hematological and/or tissue sampling, physicochemical data, or subject-specific metadata. This will strengthen the model and make it more accurate.

Other inputs to the model may be data related to one or more of the following: prosthetic heart valves, vascular devices or grafts.

For the purpose of the simulation using the provided subject-specific computational model according to the invention, the fluid can be modelled as either incompressible, compressible or weakly compressible, the fluid can be modelled as either Newtonian or non-Newtonian. The invention is not limited by the choice of fluid rheology and compressibility.

Simulations using the generated model may be demonstrated on, but not limited to, a holographic 3D screen or by the use of augmented or virtual reality, thus visualizing possible alternative treatments. The presentation can potentially be augmented by merging of data, such as combining structural information with functional data in a parametric fashion.

As an example, a 3D screen is available during surgery, and the data it displays may be streamed real-time from the echo machine. Immediately after valve repair, the results are tested with pre-surgery simulation. When the result is not satisfactory, a correction of the repair or valve replacement may be performed.

Before leaving a hospital, a last echocardiographic recording is performed and will form the basis for later controls. Controls will be an important part of the workflow for evaluation of short- and long-term results.

The disclosure thus enables an optimized assessment of the outcome in cardiovascular disease, and offers system for a continuously assessment by:
- Assembling all pre- and post- operational data and experiences and gather these in the database;
- Improving all process steps bases on pre- and post- operational knowledge, history matching and other knowledge;
- Continuously performing better both with respect to medical outcome as well as technically designing grafts and valves based on patient-specific data.

### Method for subject-specific computational models

The method for providing a subject-specific computational model of at least one component in the cardiovascular system can be realised and implemented in different products used in a process for clinical treatment planning and/or diagnostic purposes, in pre-, peri- or post-operative decision support using the subject-specific computational model obtained by the inventive method. These products and the resulting services will comprise:
1. A subject-specific simulation model of a pumping heart, able to simulate the complex flow phenomenon and provide flow details. The model is obtained from medical imaging data, such as ultrasound, magnetic resonance imaging (MRI) and computed tomography (CT) and flow measurements and may be used for 2D, 3D and 4D flow simulations. The model is preferably subject-specific, i.e. based on data obtained from each individual.
2. Workstation for processing of medical data, images and vascular disease knowledge as input for generating the subject-specific computational model and using this for optimized patient understanding aimed at optimal diagnostics and treatment including a prediction of the best outcome for each patient.
3. Service provided for patients, insurance companies including their subsidiaries, clinicians, cardiologists, surgeons, medical physicists, engineers and other users of image-based products and interpretation. The service provides optimized diagnostics and treatment including a prediction of the outcome of a medical procedure for each patient by using the subject-specific computational model.
4. Individually prosthesis designs of grafts, valves and other cardiovascular devices for surgical procedures, which are based on data obtained from simulations using the subject-specific computational model for designing optimized individual prostheses designs.

### Subject-specific models:

The disclosure regards models and methods that are able to contribute to a better understanding of the hemodynamics in the heart. To obtain physiological realism the models are preferably subject-specific models.

The disclosure includes a geometry-prescribed CFD approach for the heart chambers. To achieve subject-specific boundary conditions, the invention uses geometries obtained from medical imaging data. The medical imaging data used by the invention may be ultrasound (or echocardiography), magnetic resonance imaging (MRI) and computed tomography (CT). Cardiovascular medical imaging, in particular ultrasound, MRI and CT, has reached a level that provides high quality geometrical data. Today, ultrasound (or echocardiography) is the leading imaging tool in cardiology. A major benefit of ultrasound (or echocardiography) is that it allows for real-time inter-active display of image data and can therefore help in guiding treatment.

The models provided by the invention may be based on medical data acquired from different imaging modalities such as Magnetic Resonance (MR), X-ray, Computational Tomography (CT), Positron Emission Tomography (PET) and ultrasound (e.g. ultrasonography, echocardiography) for both 2D and 3D flow simulations. However, other imaging modalities may also be used.

The mitral valve has a complex three-dimensional geometry and movement pattern. The two mitral leaflets are thin, rapidly moving structures, which undergo large deformations during a heart cycle. The modelling of the mitral valve is therefore a challenging task and currently, no single method solves this task completely. Due to the complexity, the mitral leaflets are often excluded in simulations of ventricular flow. By use of an FSI algorithm able to handle two asynchronously moving, rigid leaflets, the invention enables study of how the mitral leaflets affect the intraventricular flow field during diastole. An advantage of the invention is that this enables to avoid the use of symmetry in the simulations. When the anterior and posterior valve lengths are based on ultrasound (or echocardiography) recordings, the invention enables analysis of the leaflets' impact on the flow field in a 2D simulation of ventricular filling.

Due to the complexity of the heart, the left atrium (LA) is often neglected in simulations of LV filling. The LA is then replaced by some simplified inlet condition, like a uniform pressure condition or some symmetric velocity profile. Relatively few studies have focused on the normal flow distribution inside the LA and the understanding of the global flow pattern within the atrium is therefore sparse. The model as supplied by the invention allows for investigation of the flow inside the atrium and the resulting velocity distribution at the mitral orifice. Both 2D and 3D simulations are used for this purpose. As such, the filling simulations of the invention gain knowledge of inlet conditions to the LV.

The present disclosure is a subject-specific CFD model, which is generated from medical imaging data and flow measurements, and they may be used to investigate healthy and pathological cardiac blood flow and to simulate the effect of virtual surgery and thereby optimize treatment.

The disclosure is the first subject-specific 3D CFD model based on real-time 3D echocardiography (RT3DE). The model may be based on a surface-tracking method of the heart chambers from 3D echocardiographic data. The 3D CFD model of the invention may include a physiologically representation of the mitral valve.

The simulation model of the disclosure includes a system enabling construction of 3D CFD models based on data from RT3DE found in ultrasound scanner systems. Such real-time CFD simulations have the potential to improve and change clinical practice.

In one aspect of the disclosure, the simulation model may be used to choose the optimal rotation position of a valve (either artificial, mechanical or bio-prosthetic valves) based on the flow dynamic for each individual. In this case, data related to prosthetic heart valves and/or devices are input data to the model. This may reduce the risk for calcifications or other factors influencing the need for reoperation.

In another aspect of the disclosure, the model is created with input data related to corrective surgical procedures with the aim of correcting the natural components or part of the heart, i.e. bulging heart valves, para-valvular leakage etc.

In one aspect of the disclosure, the model is created by inputting data from both subject-specific and non-subject-specific data of the cardiovascular system and components thereof, where the non-subject-specific data represent data being applicable to many individuals. These data can be input to the model prior to generating the model or during generation of the model for further optimisation.

In one aspect the model provided according to the invention can be arranged as a machine learning model for continuously optimizing treatment planning and/or decision making and/or for diagnostic purposes by inputting at least one of the following: prior simulation results, patient history, and pre-, peri- or post-operative effects. The machine learning will then, based on experience and outcome of earlier results learn and improve without being explicitly instructed.

By applying a machine learning model, the model itself is able to choose which procedure to simulate based on on one or more of the following: suggestions from the machine learning system; information of different cardiovascular devices and choices made by personnel and/or the patient.

### The workstation:

In addition to the collection of various data as mentioned above, the workstation may further comprise means with statistical data and options for history matching. This provides a basis for optimal diagnostics and choice of treatment more objective and reproducible than otherwise possible.

The station can for example be implemented in the operating theatre, in the cardiologist office or for use during the pre-operational meeting with the whole team or parts of the team. The development can be aimed also towards implementation in existing or new imaging modalities.

### The service provided:

1. Collection of patient data.
2. Analysing the data from the previous step.
3. Combining the data from step 1 with statistical data and history matching in such manner that new data can be generated which can include pressure, flow velocity, wall shear stress, wall stress, wall strain and other flow-, tissue- and electrical parameters.
4. The outcome will be a description of the new knowledge and understanding based on the data in step 3.
5. The description will contain a more optimized and objective diagnostics and treatment of the given patient.
6. The description will be sent back to the parts concerned.

The service may be provided internally from the hospital or by an independent company.

### The individualized prostheses design:

Information obtained from the disclosure such as obtained from the provided simulation model, from the workstation and / or the service, provides a basis for designing patient specific/individualized grafts and / or valves. The new individualized grafts/valves will be returned to the simulation model / workstation /service to perform a pre-operational simulation/analysis of the post operational effect. This is done to decrease the number of re-operations and medication and increase long-term survival and quality of life. The grafts / valves can be made of different materials like metals, polymers, composites, ceramics, biological materials (including stem cell based development) etc. in such a way that shear stress, flow pattern, tissue stress and overall functionality will be optimized for long term survival and optimal quality of life. The grafts/valves may be produced using additive manufacturing (3D printing) or casting/moulding.

### Subject-specific models based on medical imaging

Different imaging modalities like CT, MRI and ultrasound (or echocardiography) have in recent years been supported by simulation tools. In most numerical studies, MRI has been used to obtain the transient geometries. MRI has a clear benefit with respect to image quality and has the advantage of producing anatomically detailed and functionally accurate datasets. MRI has also been a 3D method since its beginning. A drawback, however, is that the cardiac valves are less distinguishable due to high signal from blood.

To obtain the model according to the invention, the reconstruction of a 4D (3D + time) volume is achieved over multiple heart cycles something, which requires long acquisition time and the need for respiratory gating. However, a long acquisition time increases the cost of the examination and inter-slice alignment errors might occur due to different diaphragm positions in subsequent breath holds. In addition, MRI recordings cannot be performed on people with metallic implants, like some artificial heart valves. In such cases, MRI might not be feasible for all model building purposes. Due to high cost and complexity, the use of MRI might also be restricted on cardiac patients. As an alternative method for obtaining the invention, the inventors thus propose the use of cardiac ultrasound to build the simulation model.

Cardiac ultrasound, often referred to as echocardiography, is, among medical doctors, the most applied method for diagnosing the heart. The particular strength of ultrasound is its ability to record moving structures in real-time and it can therefore be used to help guide invasive procedures. It is also a relatively easy and cost-effective imaging technique. Another important advantage of echocardiography, which is highly valuable in respect of this invention, is the clear visualization of the cardiac valves. Echocardiography may yield a larger inter-subject variation in image quality than MRI, but is still most feasible in regards to model building purposes.

2D ultrasound has been on the market for several decades. 3D ultrasound, on the other hand, was first introduced by Philips in 2002. Since then, other providers of ultrasound systems have released systems with real-time 3D capabilities. 3D ultrasound has undergone large improvements the last few years, and both image quality and temporal resolution are now at a level that makes it possible to extract high quality 3D geometries and deformations. There exists an extensive amount of 2D echocardiographic patient data. Thus, 3D echocardiography is gaining popularity as a routine clinical tool.

All types of medical imaging, along with flow measurements may be applied for building models according to the invention. In one embodiment of the invention, the models are based on cardiac ultrasound (or echocardiography). The extensive amount of such ultrasound patient data available makes this embodiment particularly useful. In addition, ultrasound (or echocardiography) is the major imaging tool in cardiology. Furthermore, developing models from ultrasound data instead of MRI data result in a more cost-effective, clinically viable tool with a broader area of application. If for example in-vivo recordings from patients with mechanical heart valves are necessary, ultrasound is better suited than MRI. Ultrasound also allows for real-time inter-active display of image data and flow measurements and therefore has the potential to help in guiding treatment.

### The left ventricle

Most of the computational models of the heart have focused on the LV. The earliest work was mainly generic and did not rely on subject-specific data. In the last decade, both computational and imaging resources have increased and enabled the opportunity to create more refined subject-specific models.

### The left atrium

Relatively few studies have focused on modelling the LA. While the ventricular models have gotten more refined, the models of the left atrium are still mostly oversimplified. Even if the LA provides the inlet conditions for the ventricle during diastole, the LA is most often excluded in simulations of intraventricular and transmitral flow. The atrial cavity is then replaced by an approximated inlet condition imposed directly at the mitral opening or at the end of some tube. However, the LA is far from being a passive transport chamber prior to the LV. In fact, the normal LA has important roles in optimizing left ventricular filling.

It is possible to render the atrial geometry with 4D AutoLVQ, but today, the details of the complex 3D LA geometry like the left atrial appendix and the entry locations of the pulmonary veins are not easily detectable with this software. MRI is more effective in providing detailed and complete imaging of the LA and its PVs. In their 3D study of intra-atrial flow, the inventors therefore used MRI to provide the 3D geometry of the LA.

To overcome potential uncertainties of which errors that might have an influence on the simulation results when applying the numerical simulations of the invention, the inventors have compared the simulation results with in-vivo flow measurements obtained with MR phase mapping, as MR is considered as the gold standard for non-invasive quantification of blood flow and velocity.

### Modelling the mitral valve

The mitral valve has a complex geometry and movement pattern. The two mitral leaflets are thin, rapidly moving structures which undergoes large deformations during a heart cycle. Until now, no single method has been applicable to every problem. To find the method that is best suited, one has been forced to focus on the features of the given problem. If the interest lies only on the mechanical behaviour of the valve, the interaction with the fluid flow does not have to be considered. If the focus is purely on the fluid dynamics, then the motion of the leaflets can be prescribed, e.g. from experimental data or medical imaging data. If it is important to have flow driven leaflets, an FSI approach is necessary.

Another aspect is whether the problem requires rigid or flexible heart valves or if a subject-specific model is desired. Most of published heart valve models have focused on fluid motion near a mono-leaflet or bi-leaflet mechanical valve in a steady flow [66]. A subject-specific model of the mitral valve is, on the other hand, a very challenging task. A subject-specific FSI model requires subject-specific material parameters, something which has been difficult or even not possible to obtain for a heart valve. A subject-specific geometry-prescribed model requires detailed information of the valve dynamics from medical imaging data, currently such information has been difficult to obtain. Technical advances have enabled echocardiography to identify valve structures and the time resolution in echocardiographic recordings is also sufficient enough to capture the leaflets' motion. However, due to their rapid movement, the time-dependent shape of the valve leaflets is not easily extractable. There does not exist any automatic tool for valve segmentation, thus, manual tracking is the only alternative available today.

Previously the inventors have focused on flow driven rigid leaflets, and they have simulated two rigid, asynchronously moving mitral leaflets during ventricular filling. For that purpose, they used the partitioned FSI technique. Because the leaflets strongly interacted with the surrounding fluid, an implicit coupling scheme was necessary to achieve equilibrium between fluid and structure. The implicit coupling scheme, as presented by the invention, is an extension of the coupling scheme for one leaflet developed in Vierendeels et al. and validated in Dumont et al. The FSI algorithm, supplied by the invention, is tested in a 2D simulation, where the mitral valve was rendered as two rigid asymmetric leaflets with lengths obtained from ultrasound (or echocardiography) recordings. The algorithm applies to 3D structures as well.

The inventors have also focused on the fluid dynamics in a subject-specific 3D model of the LV. The prescribed ventricular boundary conditions were obtained from RT3DE. A physiological representation of the MV was also desired in this 3D model. Because 3D tracking of heart valves from medical imaging data is a complicated and time-consuming task, the inventors used an advanced 3D finite element material model to represent the MV geometry. A transient simulation of the FE MV model in Abaqus provided us with the time-dependent systolic movement of the valve. This prescribed valve motion was subsequently implemented as a boundary condition in the 3D CFD model. The FE MV model is not subject-specific; however, the valve model can be modified to follow different subject-specific valve profiles, e.g. rendered from 2D echocardiographic images.

In addition, a study on how the curvatures of the mitral leaflets influence the systolic flow field is also executed. In that study the inventors focused on full control of the valve motion, therefore prescribed leaflet dynamics were used. The study was first performed in 2D, then in 3D. In the 2D study, two different types of leaflet curvatures were simulated, and the resulting ventricular flow fields were compared. For the first model, normal, healthy leaflet dynamics were desired. Hence, a code for tracking structures in 2D ultrasound images was written and used to obtain the systolic motion of the valve in a healthy subject. For the second model, a more irregular valve curvature with a higher degree of billowing was studied. For this purpose, the transient cross-sectional valve profile of the 3D FE MV model, ref. *[*V. Prot and B. Skallerud. Nonlinear solid finite element analysis of mitral valves with heterogeneous leaflet layers. Computational Mechanics, 42(3):353-368, February 2009.] was implemented as a boundary condition in the 2D simulation. An initial 3D CFD simulation was also performed. For this simulation, the inventors used a specific 3D model with a modified MV geometry.

The Anterior Mitral Leaflet curvature affects left ventricular outflow as described in the following. The mitral valve (MV) connects the left atrium (LA) and the left ventricle (LV). The valve is anchored to the mitral annulus and attached to the inner wall of the LV via a series of strings called chordae tendineae. The valve assures unidirectional blood flow between the two heart chambers. During diastole, the soft leaflets fold into the LV to allow blood to pass freely. During systole, the ventricle contracts and the valve closes to prevent blood from flowing back into the LA. The valve consists of two leaflets; the anterior mitral leaflet (AML) and the posterior mitral leaflet (PML). The AML is connected to the posterior wall of the aorta, whereas the PML is connected to the ventricular wall itself. The AML is the longest in length and has the greatest motion in comparison to the PML. The movement of the PML is more restrained by the tendinous cords and the leaflet has a more supporting role during the cardiac cycle.

During ventricular contraction, a normal, healthy mitral valve billow slightly into the LA, [J. B. Barlow. Perspectives on the mitral valve. F. A. Davis Company, 1987]. There are two major components contributing to the leaflets' systolic curvature. The first, and most obvious, is leaflet billowing, the second, and subtler, is the annular saddle shape, [I. S. Salgo, J. H. Gorman, R. C. Gorman, B. M. Jackson, F. W. Bowen, T. Plappert, M. G. St John Sutton, and L. H. Edmunds. Effect of annular shape on leaflet curvature in reducing mitral leaflet stress. Circulation, 106(6):711-717, 2002*.].* The annular non-planarity and the slightly billowing leaflets in the normal, healthy MV act together to optimize leaflet curvature and thereby reduce the mechanical stress in the mitral leaflets, *[*T. Arts, S. Meerbaum, R. Reneman, and E. Corday. Stresses in the closed mitral valve: A model study. Journal of Biomechanics, 16(7):539 - 547, 1983. ISSN 0021-9290*; Salgo et al., 2002*].

While there has been an increased interest in understanding how leaflet geometry, and in particularly the systolic leaflet curvature, affects leaflet stress distribution; (*Salgo et al*., *2002*), there has been less focus on how leaflet geometry affects the blood flow; [John-Peder Escobar Kvitting, Wolfgang Bothe, Serdar Göktepe, Manuel K. Rausch, Julia C Swanson, Ellen Kuhl, Neil B. Jr Ingels, and D. Craig Miller. Anterior mitral leaflet curvature during the cardiac cycle in the normal ovine heart. Circulation, 122(17):1683-1689, 2010*.].* The inventors have found that the natural curvature of the leaflets also optimizes the flow dynamics. *Kvitting et al. (2010)* investigated the AML curvature in ovine hearts during the cardiac cycle using radiopaque markers and videofluoroscopy. They hypotezised that the natural mitral leaflet curvature provides an optimal shape for both systolic outflow and diastolic inflow. In light of this the inventors have investigated the hemodynamic consequences of an altered leaflet geometry.

One of the most common heart valve abnormalities is that one or both valve leaflets are bulging more than normal into the atrium during systole. Such abnormalities are often lumped together in the term billowing mitral leaflets or BML. For BML, edge coaptation is functionally normal and the condition is for the most part considered harmless. However, by obtaining a better fundamental understanding of how the valve geometry affects leaflet stress distribution and LV flow dynamics it is possible to assess the consequences of BML. As changes in leaflet curvature also occurs due to MV pathology or surgical interventions, this knowledge may be used to optimize the outcome of surgery. The application of repair techniques that change the geometry and motion of the MV has increased progressively during the past 20 years; [Hiroaki Sakamoto, Landi M. Parish, Hirotsugu Hamamoto, Yoshiharu Enomoto, Ahmad Zeeshan, Theodore Plappert, Benjamin M. Jackson, Martin G. St. John-Sutton, Robert C. Gorman, and Joseph H. Gorman 3rd. Effects of hemodynamic alterations on anterior mitral leaflet curvature during systole. The Journal of Thoracic and Cardiovascular Surgery, 132 (6):1414-1419, 2006*.]* . With the increased use of such repair techniques, a better understanding of both the structural and the hemodynamic implications of leaflet curvature is desired. This is obtained by the simulation model according to the invention.

The inventors used computational fluid dynamics (CFD) to quantify and confirm the relationship between leaflet curvature and LV flow dynamics. CFD-simulations supplies new fundamental insight by providing flow details on a level not possible by medical imaging alone. Preliminary CFD studies (Xiong et al. (2008), Dimasi et al. (2012), Dahl (2012)) indicate that the AML systolic curvature influences the LV outflow profile and thus the flow pattern near the aortic annulus, ref. [Fangli Xiong, Joon Hock Yeo, Chuh Khiun Chong, Yeow Leng Chua, Khee Hiang Lim, Ean Tat Ooi, and Wolfgang A. Goetz. Transection of anterior mitral basal stay chords alters left ventricular outflow dynamics and wall shear stress. The Journal of Heart Valve Disease, 17 (1):54-61, 2008.]; *[*Annalisa Dimasi, Emanuele Cattarinuzzi, Marco Stevanella, Carlo A. Conti, Emiliano Votta, Francesco Maffessanti, Neil B. Jr Ingels, and Alberto Redaelli. Influence of mitral valve anterior leaflet in vivo shape on left ventricular ejection. Cardiovascular Engineering and Technology, 3(4):388-401, 2072.]; [Sigrid Kaarstad Dahl. Numerical simulations of blood flow in the left side of the heart. PhD thesis, Norwegian University of Science and Technology, 2012.].

Previous CFD studies have frequently used simplified geometries in order to avoid the complexities of time-varying heart geometries. Dimasi et al. (2012) used static heart walls together with a displacement velocity at the walls to simulate the effect of the moving walls. Xiong et al. (2008) used static heart walls and a fictitious blood inlet near the apex. By using dynamic moving mesh, the invention makes accurate predictions possible throughout the entire systole.

By use of the CFD model according to the invention it is possible to further investigate and quantify in detail how the systolic AML curvature influence LV flow dynamics. Four different AML curvatures will be investigated for this purpose. The CFD-model, according to the invention, is based on Real Time 3D Echocardiography (RT3DE) recordings and uses a dynamic, moving mesh that adapts to the time-varying geometry of the heart. This model is the first subject-specific 3D CFD model of the LV based on RT3DE.

In a majority of the papers regarding hemodynamics in the LV, MRI has been used to extract the transient geometry. However, even though MRI provides high quality images, the cardiac valves are often less distinguishable due to high signal from blood. Echocardiography, on the other hand, provides a clear visualization of the cardiac valves. Due to its ability to identify valve structures, echocardiography was chosen as imaging modality in this embodiment of the invention. The 3D model includes a systolic model of the mitral valve based on the same RT3DE recordings. Thus, the model according to the invention and the models developed in Xiong et al. (2008) and Dimasi et al. (2012) are different when it comes to imaging modality, the modelling of the LV, the MV and the AML curvatures, boundary conditions and flow regime. As such, the invention represents an advantage compared to the prior art.

A better understanding of the relationship between the AML systolic curvature and the ventricular outflow is clinically useful. By providing practical insight that may facilitate the diagnosis and treatment of pathological or abnormal mitral leaflets, like BML, the invention represent a novel tool for assessment.

The simulation and validation of the flow in a subject-specific 3D model with a normal mitral valve is compared to three models with different degrees of billowing AML curvatures. Thus, the invention provides a method for analysing the hemodynamic consequences of alteration in leaflet geometry.

The invention provides a subject-specific model having the potential to support professionals in clinical decision-making by performing virtual surgery on a specific subject/patient. By doing this, new insights into the impact of alternative surgical interventions on the blood flow can be obtained for each individual patient. The present invention provides a solid and improved tool for predicting the outcome of a medical procedure by using the improved model. This may improve operative results in general for a specific patient and is a tool for designing optimized individual prostheses designs such as for instance heart valves.

A model can be tested and assessed by performing history matching, meaning that simulation results from the model is compared with history data. This can be a continuous and iterative process contributing to optimization of the model and a specific prosthesis design based on the model.

In one embodiment of the invention, the method comprises arranging the model to choose which procedure to simulate based on at least suggestions from the machine learning system, and optionally information of different cardiovascular devices including valves and choices made by a person, e.g. medical expert, professional or the patient. A chosen procedure may then be simulated for testing if the procedure is the best procedure for a specific subject or if another procedure should be chosen. The model according to the invention is further a tool suited for deciding if corrective procedures should be performed, e.g. removing, sewing or adding tissue (e.g. chords).

The model is also suited for producing optimal subject-specific 3D-designs for individual prostheses object prior to 3D-printing an object.

### Example of aspects

The algorithm is tested in a 2D simulation of the mitral valve during diastolic filling, where the valve is modelled as two rigid, asymmetric leaflets. The results indicate that important features of the diastolic flow field may not be predicted using symmetric leaflets or in the absence of an adequate model for the LA, particularly during diastasis and atrial contraction.

The algorithm applies to 3D structures as well.

Due to the complexity of the heart, the LA and the MV are often neglected in simulations of ventricular filling. However, it is important to know what impact such limitations might have on the resulting flow pattern. A qualitative investigation of the influence of left atrial inlet conditions and flow driven mitral leaflets on the diastolic ventricular flow pattern was performed. Three 2D models were created. In the reference model both the LA and the flow driven leaflets were included, while in the two other models, either the LA or the leaflets were excluded. The transient geometry of the LV was rendered from 2D echocardiographic recordings and the same wall motion was implemented in all the three models. It is important to notice that although the investigated 2D models cannot simulate the real 3D filling process, some qualitative information can be obtained.

The inventors have found that in the model where the LA and some venous inflows were included, vortices developed inside the LA during diastole. The atrial vortices caused a non-uniform velocity profile across the mitral opening, which in turn, influenced the dynamics of the leaflets and the intraventricular flow pattern. In the model where the inflow region was rendered as a tube with the inlet at the far end, no vortices were generated in the inlet geometry and the resulting mitral velocity profile was approximately uniform. Based on these observations, the inventors found that a realistic representation of the atrium should be included in simulations of LV filling and MV dynamics to achieve a physiologically representation of the velocity profile at the mitral orifice. 3D simulations are necessary for quantitative information of the intra-atrial flow field and the velocity distribution at the mitral plane.

The leaflets' influence was significant in the 2D study. The leaflets formed an inflow tract, which guided the flow into the ventricular cavity and reduced the recirculation in the aortic outflow channel due to the presence of the anterior leaflet.

An anatomically based 3D CFD model of the LA and its PVs was developed from MRI data and used to investigate the flow field during diastole. Two additional models were constructed to examine the impact of venous entry locations on the intra-atrial flow and on the resulting velocity distribution at the mitral plane. The intra-atrial flow is made up of four crossing jets flowing into an asymmetric chamber and is therefore complex. The invention illustrate that the locations of the PVs have a significant impact on the intra-atrial flow and the mitral velocity profile. The findings indicate that asymmetric located PVs might prevent instabilities in the flow field. The inventors observed that in the anatomically representative model, where the PVs were asymmetrically located, the venous jets flowed towards the mitral plane without noticeable collision. Thus, the invention provides a model, which may illustrate how different versions of PVs location have different outcome. What has been found is that one has a more uniformly distributed mitral flow profile with lower maximal velocity than the two other models where the PVs were located at the same height. The mitral plane velocity profile in the anatomically representative model, showed qualitatively good agreement with MRI flow measurements.

Due to its complexity, it is very difficult to predict the nature of the mitral jet by making general deductions about the conditions under which the jet is formed. The interpatient variability in PV number and branching patterns is large, hence, the mitral velocity profile should be considered as a subject-specific property. The invention thus provides a means to assess how variability in PV number and branching patterns influence the blood flow. This knowledge makes the invention a useful tool in pre-surgical planning and as a simulation tool applicable as an intra-surgical tool. A representative geometry of both the LA and the PVs is essential for physiological simulations of LV filling and MV dynamics. As such, the invention may influence future CFD studies regarding transmitral and intraventricular flow.

A technique for creating subject-specific 3D boundary conditions for simulations of intraventricular flow has been developed. As such, the invention provides a method for creating subject-specific 3D boundary conditions for simulations of intraventricular flow, based on subject-specific data obtainable by an imaging modality. The algorithms provide a framework for the coupling of different data sets of the LV, the LVOT and the MV.

In one embodiment of the invention, real-time 3D echocardiography was used to provide the time-dependent ultrasound images, and thus the geometry of the LV wall. As far as we know, this is the first subject-specific 3D CFD model rendered from RT3DE. Simulations performed in real-time can be based on data streamed via a data cloud.

It is difficult to obtain the 3D dynamics of the mitral leaflets from medical imaging data. A 3D FE MV model was therefore included in the LV grid topology to represent the geometry and movement of the valve leaflets. The FE MV model was pre-simulated in Abaqus, however only the systolic phase of the cardiac cycle was computed. The prescribed valve motion and the final systolic curvature can be modified to follow different subject-specific valve curvatures, e.g. rendered from 2D echocardiographic images.

To examine the correlation between this first model and the echocardiographic recordings, the model was realigned with the original echocardiographic data. A reasonable agreement was obtained.

A preliminary CFD simulation of the blood flow during ventricular contraction was performed (Appendix A in Dahl, 2012). As a first step for validation, the maximum velocity out of the LVOT was compared with in-vivo velocity measurements from MR phase mapping scans. The MR acquisitions were taken at the same day and in the same subject as the RT3DE acquisitions. Even if the velocities could not be directly compared, the comparison with the in-vivo flow measurements indicated that the results were within the physiological range.

The invention comprises use of an imaging modality for diagnosing heart valves abnormalities, such as for example billowing mitral leaflets, by obtaining subject-specific data to be applied along with fluid dynamics in a simulation model. The imaging modality may be ultrasound, MR and/or CT. Preferably it may be echocardiography. The invention is further a subject-specific computational fluid dynamic model comprising heart anatomy, in particular left atrium segment and/or left ventricle segments and/or the mitral valve. Further, the invention is directed to use of a subject-specific CFD-model in construction of artificial valves and/or grafts. A method for producing a subject-specific heart valve or graft based on information obtained from the described CFD-model is also embodied in the invention.

Further, the invention is a subject-specific model comprising numerical simulations of cardiac blood flow, and dynamic moving mesh of the heart chamber and/or the left ventricle and/or the left atrium and/or the mitral valve and /or time-varying geometry.

The following represents examples using 3D echocardiography and segmentation of the LV for providing the subject-specific computational model according to the invention.

Real-time three-dimensional (3D) echocardiography (RT3DE) (also known as four-dimensional (4D) echocardiography) with consecutive segmentation of the endocardial LV wall are the preliminary steps in building our subject-specific CFD model.

The 3D echocardiography LV volume of a 30 years old female volunteer was acquired using a Vivid E9 scanner using a 3V matrix probe with a center frequency 2.4 MHz (GE Healthcare Vingmed, Horten, Norway). The volume was acquired during apnea over 4 heart cycles, from the apical window, in harmonic mode, one QRS triggered sub-volume acquired per heart cycle. The frame rate was 27 per cycle.

For our study the endocardial border was generated using the AutoLVQ tool (Hansegård et al., 2009), EchoPAC workstation (version BT 11), GE Vingmed Ultrasound, Horten, Norway. Auto LVQ represents the LV boundary as a deformable model and relies on 3D energy minimization for evolving it. A combination of internal, external and temporal forces ensures shape continuity, while adapting the model to a particular 3D echo recording. The endocardial contour process was initialized by manual positioning of the apex and the mitral valve attachment points in a long-axis view (e.g. four chamber), both at end-diastole (ED) and end-systole (ES). After manual selection, the endocardial border is automatically generated throughout the cardiac cycle. The proposed contour was then evaluated in both short- and long-axis cut-planes of the 3D volume. If necessary, the border can be further refined by adding additional attractor points that pull the model towards the endocardium. In this case, the border was adjusted by placing a limited number of attractors. The papillary muscles and major trabeculae were included in the LV cavity.

The segmentation of the endocardial LV wall resulted in 27 closed three-dimensional surface meshes, one for each timeframe. Each of the 27 meshes consisted of 1946 nodes and 3888 triangular cells.

Fig. 3 shows a closed 3D surface mesh of the endocardial LV at one instance of the cardiac cycle. The resulting meshes were exported and used to reconstruct the LV geometry and systolic movement.

The focus in this study is on the systolic part of the heart cycle from the onset of aortic valve (AoV) opening to AoV closure in end-systole, i.e. the isovolumetric contraction in start-systole is not included. The length of this periode in the specific heart was measured to 285ms in a heart cycle of 962ms. The frame rate obtained from the RT3DE recordings was 27 for the whole cycle, where 9 frames belonged to the phase of interest, i.e. from AoV opening to AoV closure. The start geometry was reconstructed from the segmented LV wall at AoV opening.

The following describes the geometric reconstruction of the subject-specific mitral valve and ascending aorta.

The segmented LV wall, obtained from AutoLVQ, does not include the MV or the aorta. The inventors therefore had to reconstruct the physiological MV and ascending aorta (Aao) from the same RT3DE recordings as for the LV wall and include them into the original segmented LV surface mesh. An in-house software was written for this purpose.

The MV geometry was reconstructed from its physiological shape at peak systole and set to be static throughout the simulation.

Fig. 4 shows a subject-specific 3D model of the physiological mitral valve at peak systole. As seen in the figure, the curvature of the normal, healthy mitral leaflets is approximately flat at peak systole.

The ascending aorta is the first section of the aorta, commencing at the upper part of the LV base. The shape and tilting angle of the Aao was traced in the recordings and attached to the LV. During the simulation the Aao will deform in accordance to the LV base. The aortic root with its sinuses of Valsalva, which is the first part of the Aao, was simplified to a tube. The length of the Aao was set to minimize the influence of the outflow conditions on the flow field of interest.

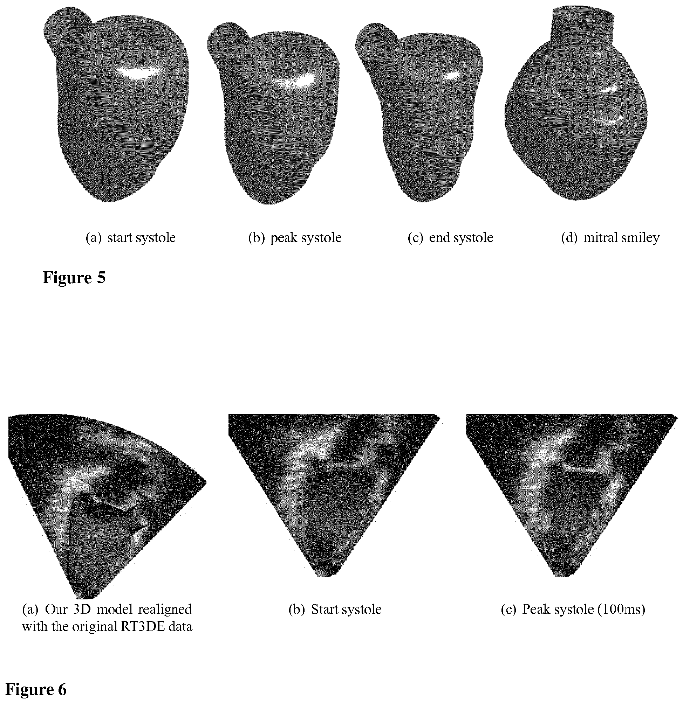

Figure 5 shows the complete subject-specific 3D model, *Cₛ*, of the LV including the MV and the proximal part of the Aao at different instances during a complete systole. Figure (a) is from start systole, (b) is from peak systole, i.e. 100ms into the simulation from AoV opening, and (c) is from end systole (285ms). Figure (d) shows the model from an atrial view, where a mitral "smiley" is clearly visible.

The time-varying 3D endocardial surface mesh obtained from AutoLVQ was used to create the prescribed subject-specific LV movement throughout systole. The surface meshes from AutoLVQ consisted of 1946 nodes and 3888 triangular cells each. However, a refined surface mesh was required to obtain a reasonable accuracy in the CFD simulation. A refined mesh will result in new intermediate nodes in the start geometry which are not a part of the original mesh. This means, their nodal positions are unknown for the subsequent time frames. The nodal translations of the new refined mesh had to be interpolated from the original segmented mesh. This was achieved by using barycentric coordinates as described in Dahl et al. (2011).

The time step in the CFD-simulation of the invention is significantly smaller than the time step between the recorded frames. This required new intermediate meshes to be calculated between the segmented time frames at every CFD time step. According to the invention, this was done by spline interpolation.

In the CFD simulations according to the invention, the prescribed LV wall movement was implemented as a FLUENT User-Defined-Function (UDF) and used as a boundary condition. The Aao was set to deform in accordance to the LV, whereas the MV was set to be static throughout the simulation.

To verify the geometry of the transient subject-specific 3D model, the model was realigned with the original RT3DE data at each time-frame throughout systole, as shown for one time frame in Fig. 6 (a). The yellow line in (b) and (c) shows the contours of the 3D model in the belonging ultrasound image at start and peak systole, respectively. As can be seen in Fig. 6, a reasonable agreement between the modelled geometry and the RT3DE data was obtained.

### Modification of anterior mitral leaflet curvature

In order to investigate how the AML curvature affects the flow field, three new models were created. The models were distinct when it came to the degree of billowing of the AML.

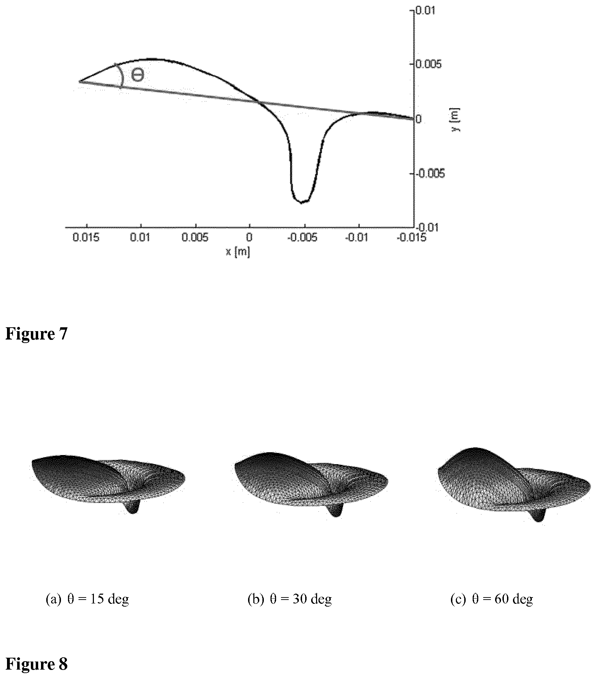

Figure 7 illustrates the AML curvature with a defined angle θ. The subject-specific 3D model, Cs, was modified by adjusting the degree of billowing of the anterior leaflet. We chose to define the degree of billowing by the angle θ. First, a line was drawn from the annulus at the anterior side to the annulus at the posterior side in the apical long-axis view, as shown with a dotted line in Fig. 7. Then, the angle θ of the AML curvature was measured from this line as indicated in Fig. 7.

The angle of the subject-specific AML curvature in model *Cₛ*, was measured to -2 degrees. The three modified models were created with angles θ of 15, 30 and 60 degrees and will be referred to as C₁₅, C₃₀ and C₆₀, respectively. The three MV models are shown in Fig. 8.

A dynamic tetrahedral mesh was used in the simulations. The mesh size varied between the different cases between 768 - 920 k cells. The geometry of the LV is smooth with the exception of the coaption zone between the anterior and posterior leaflets and the transition from the anterior leaflet into the aorta. The largest velocity gradients can also be found here, thus sizing functions where used to concentrate the mesh in regions with large velocity gradients. The sizing functions specify a lower and upper bound on the mesh size in the model, and a growth rate between successive cell sizes. In this study, we used a lower and upper mesh size of 0.3 mm and 3.0 mm, respectively. A 20% change in cell size was allowed. The sizing functions where attached to the mitral valve and the ascending aorta. Thus, the grid resolution would be highest in the regions with the highest gradients. A mesh dependence study was performed on the subject-specific model investigating the effect of cell size on the calculated solution. It was found that reducing the initial mesh size below 0.3 mm had little effect on the attained solutions, e.g. using an initial mesh sizes of 0.15 mm did not change the flow field appreciably (as judged from calculated distribution of wall shear, pressure and velocity profiles). The time step was adjusted so that the CFL number was less than 0.1.

Flow simulations, using the subject-specific model provided by the inventive method, were performed using the commercial finite volume package Ansys Fluent 15.0 (Ansys Inc.). The CFD solver was extended with dedicated UDFs in order to include the systolic movement of the 3D model in the simulations. The prescribed subject-specific wall motion drives the flow. The ALE formulation was used to express the Navier-Stokes equations on the moving grid. Because we use prescribed wall motion we only need to compute the pressure gradients rather than the absolute pressure, this is appropriate since a pressure gradient is a relative and not an absolute variable. The base pressure can be set to any value, as it will not influence the hemodynamics, in our simulations the base pressure was chosen to be zero. However, the absolute pressure is important if one would like to estimate the hemodynamic work or calculate fluid structure interaction.

The simulation starts from the onset of the aortic valve (AoV) opening and ends at AoV closure. The length of this period was 285ms and given from the RT3DE recordings. Laminar flow was assumed. The blood was modelled as an incompressible, Newtonian, homogenous fluid, with a density of ρ = 1050 kg/m³ and a viscosity of 3.5 · 10⁻³ kg/ms, which are reasonably good approximations for blood flow in large cavities (Ku, 1997). A no-slip condition was imposed at the walls. The fluid was assumed to be at rest at the time of AoV opening.

When discussing the general features of the flow field one should keep in mind that the flow is highly transient, and that the timescales involved are rather small (the systole lasting 285 ms for the subject considered in this study). There are no periods of steady or quasi-steady flow during the systole. The flow is either accelerating or decelerating with peak systole separating the two regimes. The bulk flow is actuated by the contraction of the heart muscle causing the ventricle walls to move changing the volume of the ventricle. During the early contraction the volume does not change much, and pressure builds up in the ventricle until it exceeds the end diastolic pressure in the aorta which in general lies in the range of 80 mmHg. Once the pressure inside the ventricle exceeds the end diastolic pressure in the aorta the aortic valve opens and the flow inside the ventricle and aorta starts accelerating due to the pressure gradient produced by the contraction of the heart. In the present work, the fluid is assumed to be at rest when the aortic valve opens. Substantial effort has been devoted to characterizing the flow in the ventricle during diastole, and it is clear from this body of work that complex flow structures will exist in the ventricle at beginning of the systole. However, in order to understand the overall flow features produced by the ventricle during systole we believe our assumption serves as a good starting and reference point for assessing flow behaviour.

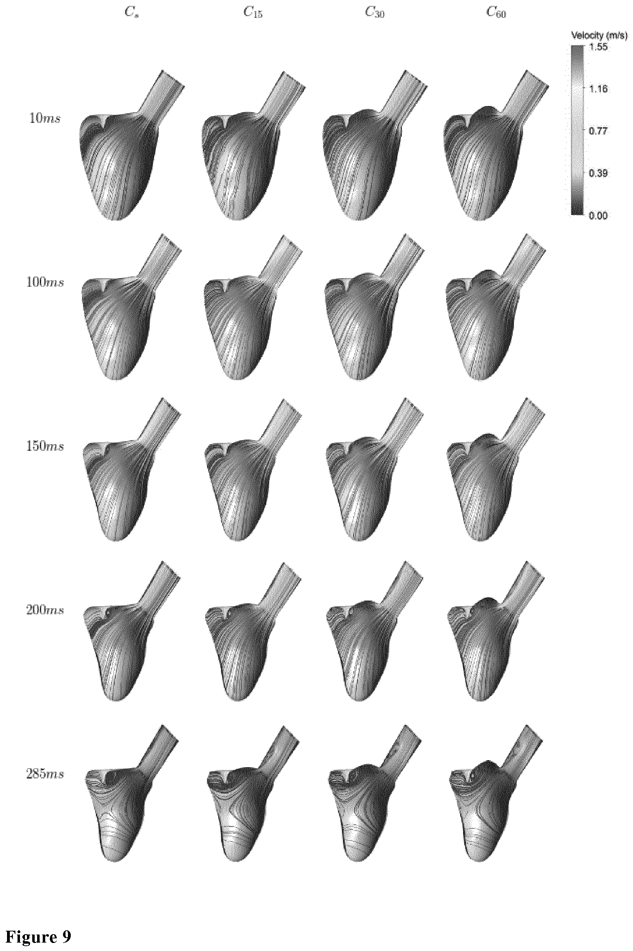

The overall flow is one from the apex towards the aorta. Fig. 9 shows velocity streamlines in a long-axis view of the LV at 5 different time steps, i.e. 10, 100, 150, 200, 285 ms. The four columns are Cₛ, C₁₅, C₃₀ and C₆₀, respectively. Global velocity ranges from 0 to 1.55 m/s.

The streamlines starting out as perpendicular to the walls and then flowing out of the aorta being parallel to the aortic wall. The streamlines in Fig. 9 are coloured based on the magnitude of the flow velocity. As can be seen the flow accelerates appreciably as it moves through the contraction represented by the aorta. The flow around the coaption zone of the MV and the billowing of the AML can be seen to perturb the streamlines. We will discuss these flow features in turn below.

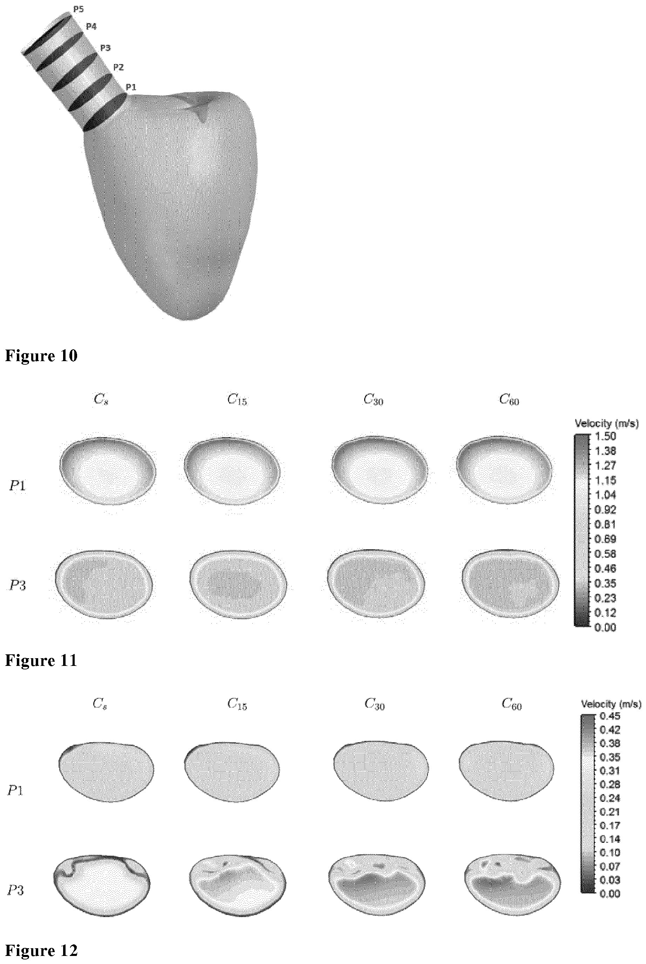

Figure 10 illustrates the location of the cross sections P1 to P5, where P1 is at the level of the aortic annulus, P2 is located XX cm downstream of P1. The posterior and anterior side of the ascending aorta (Aao) is also depicted in the figure.

As can be seen in Figs. 9 and 11 there is very little difference between the four cases before peak systole. In all cases we see a slightly skewed velocity profile at the entrance to aorta. However, as the flow ascends through the aorta the flow profile quickly evens out. After peak systole the retrograde pressure gradient forms (the fluid is decelerating) this combined with the strong curvature of the streamlines at the transition between the AML and Aao will result in flow separation and formation of vortical structures along the "posterior aortic wall". As billowing increases, streamline curvature increases thus so does the pressure gradient normal to the streamlines. The pressure gradient is of the order ρv²/r_{curv}, thus for a velocity of 1m/s and a radius of curvature of the streamline of say 1cm, we get an acceleration of the order 100m/s² or ten times gravity.

As can be seen from Fig. 11 there is little change in the maximum velocity in e.g. cross section P1 and P3 at any given time in the systole for all cases considered. The variation of maximum velocity with time between cross sections PI through P5 for a given case Cₛ , ... C₆₀ is much larger than the variation between cases at any given cross section. Thus, concerning the maximum velocity in any cross section throughout the aorta, billowing only has a minor influence.

However, from Fig. 12 it is apparent that billowing does affect the flow distribution and flow separation in the Aao. This is - as mentioned above - due to the curvature of the streamlines in the region transitioning from the AML to the Aao. Streamlines on the opposing side, i.e. in the transition between the ventricular wall and the Aao exhibit very little curvature and hence do little to trigger flow separation. The clinical implication of this is discussed below.

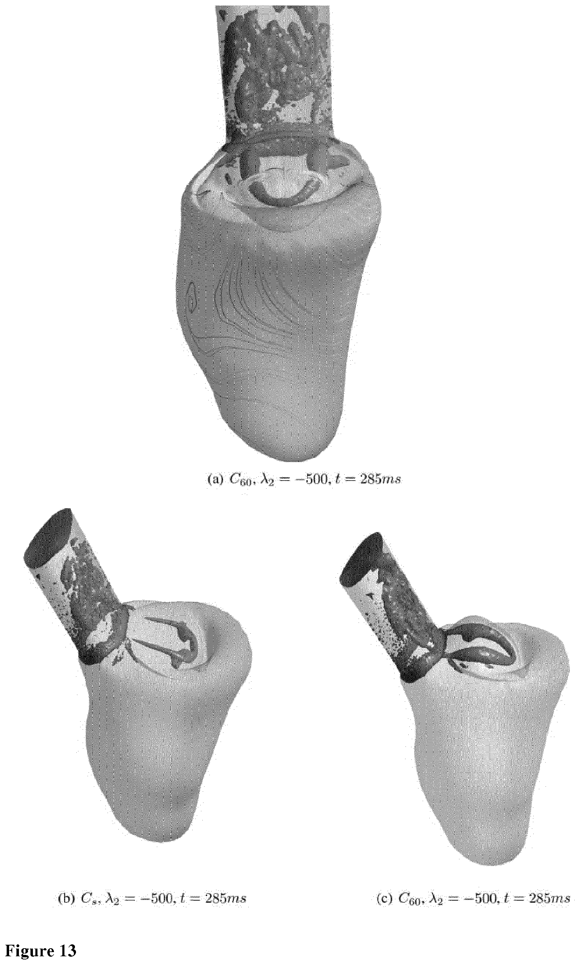

Flow features in the coaption zone of the MV and the billowing AML
is now described:
The flow produced by the ventricular wall below the attachment area of the PML is obstructed by the coaption zone of the MV. The flow is forced around the coaption zone producing a vortical structure on the AML side. This vortical structure grows with time and its ends are convected towards the aorta, creating a U-shaped vortical structure at the end of the systole as seen in Fig. 13. Figure 13 (b) and (c) depicts the vortical structure for case Cₛ and C₆₀, respectively at the end of the systole just prior to closure of the aortic valve. Fig. 13 (a) depicts the same case as in Fig. 13 (c) but from a different view angle and with the streamlines in an apical plane (90°) to illustrate how the flow swirls around the vortex core. The vertical structures have been visualized using the so called λ₂ criterion (Jeong and Hussain, 1995). The λ₂ criterion is based on the second eigenvalue of the tensor S² + Ω² where S and Ω are the symmetric and anti-symmetric parts of the velocity gradient tensor ∇ 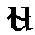 . The second eigenvalue, λ₂, of this tensor, S² + Ω², has been proven to correctly capture the pressure minimum of vortical structures in both turbulent and low Reynolds number transient flows. AML billowing tends to enlarge the vortex size. Streamline curvature and contraction at the aortic root and the retrograde pressure gradient responsible for flow separation in the aorta also result in vortical structures forming in the aorta at the end of the systole. The development of the vortical structures for Cₛ and C₆₀ are shown in Fig. 13. As can be seen vorticity is produced at the tip of the coaption zone until the late systole when the retrograde pressure gradient forms and flow in the region is reduced.

To control whether the CFD model provides results within the physiological range, an initial validation of the 3D model was performed by comparing the CFD results with flow measurements obtained from MR phase mapping scans of the same heart recorded at the same day as the RT3DE acquisitions. The simulated velocities were in accordance to the measured flow data and indicated that model according to the invention provides results that are within the physiological range.

The CFD results were also validated by using approximate analytical methods, using the continuity and Bernoulli equations to estimate velocity and pressure.

### Clinical perspective

Based on the present study we believe that AML curvature does not substantially change the overall performance of the heart as a pump. Only minor losses in efficiency could be identified and calculated. Furthermore, only minor changes to the overall flow pattern were found. Only one of these changes do we believe to be of physiological importance, and that is that of stream- line curvature at the aorto-mitral junction. As AML curvature increases streamline curvature will increase in this region. Our calculations show that the pressure gradient formed by these curved streamlines enhance flow separation in this region. The aortic valve is comprised of three leaflets. The net lift on each of these leaflets is the result of a force balance comprising of static pressure difference across the leaflet, frictional forces and forces due to streamline curvature. If the first two are assumed to be independent of AML-curvature, changes due to streamline curvature could influence the dynamics of the leaflets. Based on our previous observation that there is an inherent asymmetry in the problem due to the geometric contraction as the flow enters the aorta, AML curvature (i.e. billowing) would exacerbate this asymmetry. Streamline curvature would create a region of lower pressure on the ventricular side of the leaflet, which could result in earlier closure of the leaflet(s?) close to the aorto-mitral junction. The high velocity flow would also have a tendency to attach to the surface of this leaflet which could also alter the dynamic behaviour of the leaflet and its function. We hypothesize that AML-curvature through these mechanisms has the potential to adversely affect the function of the aortic valve. Whether this is through re-modelling of the cellular structure in response to altered stress and strain, or due to altered transport processes in the fluid flow is too early to conclude.

The vortical structure formed by the flow around the tip of the coaption zone of the mitral valve could influence the coaption of the anterior and posterior leaflets. Streamline curvature could influence the force balance on the coaption zone. Changes in systolic LVOT flow pattern secondary to bulging of the anterior mitral leaflet can hypothetically cause energy loss and less efficient ejection, remodelling of the mitral leaflet due to local shear forces acting on the bulging mitral valve and lastly, disturbed aortic flow profile and recirculation jets located in the proximal part of the ascending aorta. The energy loss is trivial based on our simulations. We can only speculate whether increased shear forces are important or relevant in clinical situations with bulging of the mitral valve. This is essential after mitral valve repair for instance and may explain repair failures several years after surgery. Furthermore, an association between elongation of the anterior mitral leaflet and bicuspid aortic valve has been reported. Our simulation demonstrates increased recirculation jets located to the proximal part of the ascending aorta. In patients with bicuspid aortic valve this may theoretically accelerate aortic sclerosis. Follow up studies applying CFD-models might reveal whether these are important mechanisms. Furthermore, this new approach may also help us to predict later events.

Heart valves are active structures that respond to altered load patterns by remodelling (Grande- Allen, 2004). Although remodelling may be initiated as adaptations to altered loads, the resulting valve tissues may not be able to provide normal long-term function. Because valve tissue has this ability to remodel, the relationship between the valve geometry, the tissues microstructure, material properties and loading environment is interdependent.

### Summary

Like most of the biomechanical problems, the invention covers multidisciplinary subjects and involves scientific computing, mathematical modelling, fluid dynamics, structural mechanics and physiology. The invention provides different frameworks for providing a subject-specific computational model of at least one component in the cardiovascular system for simulating blood flow and/or structural features. This model can for instance be used for simulating the hemodynamics in the left heart. Thus, the invention contributes to computational methodology for cardiac modelling. The main findings and developments embodied in the invention are providing an implicit coupling algorithm for the partitioned FSI simulation of two rigid leaflets. The algorithm allows for asynchronous motion of two leaflets and is therefore suitable for simulating bi-leaflet mechanical heart valves. The mutual interaction between the leaflets can be accounted for in the coupling iterations by including the full 2 × 2 Jacobian matrix. The differences in convergence rate of the full Jacobian matrix versus the diagonal Jacobian matrix were compared. The results show that including the full Jacobian matrix in the coupling iterations significantly enhanced the convergence rate and thereby the speed of the simulation. Overall, the total computational time was reduced by 22.5%.

## Claims

1. A computer implemented method for providing a subject-specific computational fluid dynamic model (CFD) of at least one component in the cardiovascular system for simulating blood flow and/or structural features, wherein the model comprises transient geometry and is created by:
- acquiring subject-specific measurement data of said at least one component, and
- generating the computational fluid dynamic model based on the subject-specific measurement data, **characherized in**
- acquiring the subject-specific measurement data by performing echocardiography in real-time 3D for creating time-dependent ultrasound measurements representing medical imaging data, and
further defining at least one boundary condition or source term for the model from the transient geometry based on time-varying geometrical shapes of the medical imaging data when running a simulation.

2. The computer implemented method according to claim 1, further comprising using flow and/or pressure measurement data for acquiring flow and/or pressure specific data related to the at least one component in the cardiovascular system.

3. The computer implemented method according to claim 1 or 2, where the at least one boundary condition or source term is generated by using time-dependent movement of the at least one component in the cardiovascular system, thereby determining a movement interval of the at least one component in the cardiovascular system.

4. The computer implemented method according to claim 3, by using the time-dependent movement of at least a part of a cardiac wall, cardiac volume, cardiac or prosthetic valves as the at least one or more components in the cardiovascular system for generating the at least one boundary condition or source term.

5. The computer implemented method according to claim 3, by using the time-dependent movement of at least a part of a vascular wall, vascular volume or vascular valves for generating the at least one boundary condition or source term.

6. The computer implemented method according to claim 3, by using the time-dependent movement of at least a cardiac pumping device as the at least one or more component in the cardiovascular system for generating the at least one boundary condition or source term.

7. The computer implemented method according to any of the previous claims, where the at least one component in the cardiovascular system is a heart component.

8. The computer implemented method according to any of the previous claims, by letting the boundary conditions change dynamically during a simulation cycle.

9. The computer implemented method according to any of the previous claims, by letting the source terms change dynamically during a simulation cycle.

10. The computer implemented method according to any proceeding claims, by basing the transient geometry on medical real-time imaging data.

11. The computer implemented method according to any proceeding claims, simulating the blood flow by using a Computational Fluid Dynamics (CFD) method and/or a Fluid Structure Interaction (FSI) method.

12. The computer implemented method according to any proceeding claims, simulating the structural features of the model by Computational Structural Dynamics (CSD) and/or FSI method.

13. The computer implemented method according to any proceeding claims, creating the model by adding subject-specific data, such as one or more of: hematological sampling, tissue sampling, physicochemical data, and subject-specific metadata.

14. The computer implemented method according to any proceeding claims, by creating the model by inputting data related to one or more of the following: prosthetic heart valves, cardiac pumping devices, vascular devices or grafts.

15. The computer implemented method according to any proceeding claims, by creating the model by data related to corrective surgical procedures.

16. The computer implemented method according to any proceeding claims, by collecting measurement data comprising medical data acquired from different imaging modalities such as Magnetic Resonance (MR), X-ray, Computational Tomography (CT), Positron Emission Tomography (PET) and ultrasonography.

17. The computer implemented method according to any proceeding claims, by creating the model by subject-specific data combined with non-subject-specific data of the cardiovascular system and components thereof.

18. The computer implemented method according to any proceeding claims, by arranging the model as a machine learning model for continuously optimizing treatment planning and / or decision making and / or for diagnostic purposes by inputting at least one of the following: prior simulation results, patient history, and pre-, peri- or post-operative effects.

19. The computer implemented method according to claim 18, by further arranging the model to choose which procedure to simulate based on one or more of the following: suggestions from the machine learning system; information of different cardiovascular devices and choices made by personnel and/or the patient.

20. Process for clinical treatment planning and/or diagnostic purposes, in pre-, peri- or post-operative decision support using the subject-specific computational model obtained by the computer implemented method according to any of the claims 1-19.

21. The process according to claim 20, being used for predicting the outcome of a medical procedure.

22. The process according to claim 20, being used for designing optimized individual prostheses designs.

23. The process according to claim 20, being used for designing subject-specific heart valves and/or cardiovascular devices.

## Patentansprüche

1. Computerimplementiertes Verfahren zum Vorsehen eines personenspezifischen Numerische-Strömungsmechanik (Computational Fluid Dynamic bzw. CFD)-Modells wenigstens einer Komponente im kardiovaskulären System für das Simulieren eines Blutflusses und/oder von strukturellen Merkmalen, wobei das Modell eine transiente Geometrie aufweist und erstellt wird durch:
- Erhalten von personenspezifischen Messdaten der wenigstens einen Komponente, und
- Erzeugen des CFD-Modells basierend auf den personenspezifischen Messdaten, **gekennzeichnet durch**:
- Erhalten der personenspezifischen Messdaten durch das Durchführen einer Echokardiographie in Echtzeit-3D für das Erzeugen von zeitabhängigen Ultraschallmessungen, die medizinische Bildgebungsdaten wiedergeben, und
- weiteres Definieren wenigstens einer Grenzbedingung oder eines Quellterms für das Modell aus der transienten Geometrie basierend auf in der Zeit variierenden geometrischen Formen der medizinischen Bildgebungsdaten während der Durchführung einer Simulation.

2. Computerimplementiertes Verfahren nach Anspruch 1, das weiterhin das Verwenden von Fluss- und/oder Druckmessdaten für das Erhalten von fluss- und/oder druckspezifischen Daten in Bezug auf wenigstens eine Komponente in dem kardiovaskulären System umfasst.

3. Computerimplementiertes Verfahren nach Anspruch 1 oder 2, wobei die wenigstens eine Grenzbedingung oder der wenigstens eine Quellterm erzeugt wird unter Verwendung einer zeitabhängigen Bewegung der wenigstens einen Komponente in dem kardiovaskulären System, um dadurch ein Bewegungsintervall der wenigstens einen Komponente in dem kardiovaskulären System zu bestimmen.

4. Computerimplementiertes Verfahren nach Anspruch 3, wobei die zeitabhängige Bewegung wenigstens eines Teils einer Herzwand, eines Herzvolumens oder von Herzklappen bzw. prothetischen Klappen als die wenigstens eine Komponente in dem kardiovaskulären System für das Erzeugen der wenigstens einen Grenzbedingung oder des wenigstens einen Quellterms verwendet wird.

5. Computerimplementiertes Verfahren nach Anspruch 3, wobei die zeitabhängige Bewegung wenigstens eines Teils einer Gefäßwand, eines Gefäßvolumens oder von Gefäßklappen für das Erzeugen der wenigstens einen Grenzbedingung oder des wenigstens einen Quellterms verwendet wird.

6. Computerimplementiertes Verfahren nach Anspruch 3, wobei die zeitabhängige Bewegung wenigstens einer Herzpumpenvorrichtung als die wenigstens eine Komponente in dem kardiovaskulären System für das Erzeugen der wenigstens einen Grenzbedingung oder des wenigstens einen Quellterms verwendet wird.

7. Computerimplementiertes Verfahren nach einem der vorstehenden Ansprüche, wobei die wenigstens eine Komponente in dem kardiovaskulären System eine Herzkomponente ist.

8. Computerimplementiertes Verfahren nach einem der vorstehenden Ansprüche, wobei sich die Grenzbedingungen während eines Simulationszyklus dynamisch ändern können.

9. Computerimplementiertes Verfahren nach einem der vorstehenden Ansprüche, wobei sich die Quellterme während eines Simulationszyklus dynamisch ändern können.

10. Computerimplementiertes Verfahren nach einem der vorstehenden Ansprüche, wobei die transiente Geometrie auf medizinischen Echtzeit-Bildgebungsdaten basiert.

11. Computerimplementiertes Verfahren nach einem der vorstehenden Ansprüche, wobei der Blutfluss unter Verwendung eines Numerische-Strömungsmechanik (Computational Fluid Dynamic bzw. CFD)-Verfahrens und/oder eines Fluid-Struktur-Kopplung (Fluid Structure Interaction bzw. FSI)-Verfahrens simuliert wird.

12. Computerimplementiertes Verfahren nach einem der vorstehenden Ansprüche, wobei die strukturellen Merkmale des Modells durch ein Numerische-Strukturdynamik (Computational Structural Dynamics bzw. CSD)- und/oder ein FSI-Verfahren simuliert wird.

13. Computerimplementiertes Verfahren nach einem der vorstehenden Ansprüche, wobei das Modell durch das Hinzufügen von personenspezifischen Daten wie etwa einer hämatologischen Probe, einer Gewebeprobe, von physiochemischen Daten und/oder von personenspezifischen Metadaten erzeugt wird.

14. Computerimplementiertes Verfahren nach einem der vorstehenden Ansprüche, wobei das Modell durch das Eingeben von Daten in Bezug auf prothetische Herzklappen, Herzpumpvorrichtungen, Gefäßvorrichtungen und/oder Transplantate erzeugt wird.

15. Computerimplementiertes Verfahren nach einem der vorstehenden Ansprüche, wobei das Modell durch Daten in Bezug auf korrektive chirurgische Eingriffe erzeugt wird.

16. Computerimplementiertes Verfahren nach einem der vorstehenden Ansprüche, wobei Messdaten gesammelt werden, die medizinische Daten enthalten, die von verschiedenen Bildgebungsverfahren wie etwa Magnetresonanz (MR), Röntgenstrahlen, Computertomographie (CT), Positronen-Emission-Tomographie (PET) und Ultrasonographie erhalten werden.

17. Computerimplementiertes Verfahren nach einem der vorstehenden Ansprüche, wobei das Modell durch personenspezifische Daten, die mit nicht-personenspezifischen Daten des kardiovaskulären Systems und von Komponenten desselben kombiniert werden, erzeugt wird.

18. Computerimplementiertes Verfahren nach einem der vorstehenden Ansprüche, wobei das Modell ein maschinell gelerntes Modell für das kontinuierliche Optimieren der Planung und/oder Entscheidung einer Behandlung oder für Diagnosezwecke ist, in das vorausgehende Simulationsergebnisse, die Patientengeschichte und prä-, peri- oder post-operative Effekte eingegeben werden.

19. Computerimplementiertes Verfahren nach Anspruch 18, wobei das Modell basierend auf Vorschlägen aus dem Maschinelles-Lernen-System, Informationen von verschiedenen kardiovaskulären Vorrichtungen und Entscheidungen des Personals und/oder des Patienten auswählt, welche Prozedur simuliert wird.

20. Prozess für das Planen einer klinischen Behandlung oder für Diagnosezwecke zur Unterstützung von prä-, peri- oder postoperativen Entscheidungen unter Verwendung des personenspezifischen Rechenmodells, das durch das computerimplementierte Verfahren gemäß einem der Ansprüche 1 bis 19 erhalten wird.

21. Prozess nach Anspruch 20, der für das Voraussagen des Ergebnisses eines medizinischen Eingriffs verwendet wird.

22. Prozess nach Anspruch 20, der für das Entwerfen von optimierten individuellen Protheseentwürfen verwendet wird.

23. Prozess nach Anspruch 20, der für das Entwerfen von personenspezifischen Herzklappen und/oder Gefäßklappen verwendet wird.

## Revendications

1. Procédé mis en œuvre par ordinateur pour fournir un modèle de mécanique des fluides numérique (CFD), spécifique à un sujet, d'au moins un composant dans le système cardiovasculaire pour simuler un écoulement sanguin et/ou des caractéristiques structurelles, dans lequel le modèle comprend une géométrie transitoire et est créé par :
- l'acquisition de données de mesure spécifiques à un sujet dudit au moins un composant, et
- la création du modèle de mécanique des fluides numérique sur la base des données de mesure spécifiques à un sujet, **caractérisé par**
- l'acquisition des données de mesure spécifiques à un sujet en réalisant une échocardiographie en temps réel 3D pour créer des mesures ultrasoniques dépendantes du temps représentant des données d'imagerie médicale, et en définissant, en outre, au moins une condition limite ou un terme source pour le modèle à partir de la géométrie transitoire sur la base de formes géométriques variables dans le temps des données d'imagerie médicale lors de la réalisation d'une simulation.

2. Procédé mis en œuvre par ordinateur selon la revendication 1, comprenant en outre l'utilisation de données de mesure d'écoulement et/ou de pression pour acquérir des données spécifiques d'écoulement et/ou de pression concernant au moins un composant dans le système cardiovasculaire.

3. Procédé mis en œuvre par ordinateur selon la revendication 1 ou 2, dans lequel la au moins une condition limite ou le au moins un terme source est créé(e) en utilisant un mouvement dépendant du temps du au moins un composant dans le système cardiovasculaire, en déterminant ainsi un mouvement à intervalle du au moins un composant dans le système cardiovasculaire.

4. Procédé mis en œuvre par ordinateur selon la revendication 3, en utilisant le mouvement dépendant du temps d'au moins une partie d'une paroi cardiaque, d'un volume cardiaque, des valves cardiaques ou prosthétiques comme le au moins un ou plusieurs composants dans le système cardiovasculaire pour générer la au moins une condition limite ou le au moins un terme source.

5. Procédé mis en œuvre par ordinateur selon la revendication 3, en utilisant le mouvement dépendant du temps d'au moins une partie d'une paroi vasculaire, d'un volume vasculaire ou des valves vasculaires pour générer la au moins une condition limite ou le au moins un terme source.

6. Procédé mis en œuvre par ordinateur selon la revendication 3, en utilisant le mouvement dépendant du temps d'au moins un dispositif de pompage cardiaque comme le au moins un ou plusieurs composant(s) dans le système cardiovasculaire pour générer la au moins une condition limite ou le au moins un terme source.

7. Procédé mis en œuvre par ordinateur selon l'une quelconque des revendications précédentes, dans lequel le au moins un composant du système cardiovasculaire est un composant du cœur.

8. Procédé mis en œuvre par ordinateur selon l'une quelconque des revendications précédentes, en laissant les conditions limites changer de façon dynamique pendant un cycle de simulation.

9. Procédé mis en œuvre par ordinateur selon l'une quelconque des revendications précédentes, en laissant les termes sources changer de façon dynamique pendant un cycle de simulation.

10. Procédé mis en œuvre par ordinateur selon l'une quelconque des revendications précédentes, en fondant la géométrie transitoire sur des données d'imagerie médicale en temps réel.

11. Procédé mis en œuvre par ordinateur selon l'une quelconque des revendications précédentes, simulant l'écoulement sanguin en utilisant un procédé de mécanique des fluides numérique (CFD) et/ou un procédé d'interaction fluide - structure (FSI).

12. Procédé mis en œuvre par ordinateur selon l'une quelconque des revendications précédentes, en simulant les caractéristiques structurelles du modèle par un procédé de dynamique structurelle numérique (CSD) et/ou un procédé FSI.

13. Procédé mis en œuvre par ordinateur selon l'une quelconque des revendications précédentes, créant le modèle en ajoutant des données spécifiques à un sujet, telles qu'une ou plusieurs des suivantes : prélèvement hématologique, prélèvement de tissus, données physicochimiques, et métadonnées spécifiques au sujet.

14. Procédé mis en œuvre par ordinateur selon l'une quelconque des revendications précédentes, en créant le modèle en entrant des données concernant un ou plusieurs des éléments suivants : valves cardiaques prosthétiques, dispositifs de pompage cardiaque, dispositifs ou greffes vasculaires.

15. Procédé mis en œuvre par ordinateur selon l'une quelconque des revendications précédentes, en créant le modèle par des données concernant des procédures chirurgicales correctrices.

16. Procédé mis en œuvre par ordinateur selon l'une quelconque des revendications précédentes, en recueillant des données de mesure comprenant des données médicales acquises à partir de différents modes d'imagerie tels que la résonance magnétique (MR), la radiographie, la tomographie assistée par ordinateur (CT), la tomographie par émission de positions (PET) et l'échographie ultrasonique.

17. Procédé mis en œuvre par ordinateur selon l'une quelconque des revendications précédentes, en créant le modèle par des données spécifiques au sujet combinées à des données non spécifiques au sujet du système cardiovasculaire et des composants de celui-ci.

18. Procédé mis en œuvre par ordinateur selon l'une quelconque des revendications précédentes, en agençant le modèle comme un modèle d'apprentissage automatique pour optimiser en continu la planification du traitement et/ou la prise de décision et/ou pour des fins diagnostiques en entrant au moins un des éléments suivants : résultats de simulation antérieurs, antécédents du patient, et effets pré-, péri- ou postopératoires.

19. Procédé mis en œuvre par ordinateur selon la revendication 18, en agençant, en outre, le modèle afin de choisir la procédure pour simuler sur la base d'un ou de plusieurs des éléments suivants : suggestions du système d'apprentissage automatique ; informations des différents dispositifs cardiovasculaires et choix effectués par le personnel et/ou le patient.

20. Processus pour la planification d'un traitement clinique et/ou à des fins diagnostiques dans l'aide à la décision pré-, péri- ou post-opératoire utilisant le modèle numérique spécifique à un sujet obtenu par le procédé mis en œuvre par ordinateur selon l'une quelconque des revendications 1 à 19.

21. Processus selon la revendication 20, qui est utilisé pour prédire l'issue d'une procédure médicale.

22. Processus selon la revendication 20, qui est utilisé pour concevoir des modèles de prothèses individuelles optimisées.

23. Processus selon la revendication 20, qui est utilisé pour concevoir des valves cardiaques et/ou des dispositifs cardiovasculaires spécifiques à un sujet.
